(19) Europäisches Patentamt
European Patent Office
Office européen des brevets

(11) Veröffentlichungsnummer: **0 098 600**
**A2**

(12) # EUROPÄISCHE PATENTANMELDUNG

(21) Anmeldenummer: **83106622.0**

(22) Anmeldetag: **06.07.83**

(51) Int. Cl.³: **C 07 C 43/13**, C 07 C 43/178, C 07 F 9/09, C 07 F 9/24, C 07 C 41/01, A 61 K 31/66

(30) Priorität: **06.07.82 DE 3225225**
**27.10.82 DE 3239858**

(43) Veröffentlichungstag der Anmeldung: **18.01.84**
**Patentblatt 84/3**

(84) Benannte Vertragsstaaten: **IT**

(71) Anmelder: **Max-Planck-Gesellschaft zur Förderung der Wissenschaften e.V., Bunsenstrasse 10, D-3400 Göttingen (DE)**

(72) Erfinder: **Eibl, Hansjörg, Prof. Dr., Steinweg 51, D-3406 Bovenden (DE)**

(74) Vertreter: **Weickmann, Heinrich, Dipl.-Ing. et al, Patentanwälte Dipl.-Ing. H.Weickmann Dipl.-Phys.Dr. K. Fincke Dipl.-Ing. F.A. Weickmann Dipl.-Chem. B. Huber Dr.-Ing. H. Liska Dipl.-Phys.Dr. J. Prechtel Möhlstrasse 22, D-8000 München 86 (DE)**

(54) **Neue D-Mannitderivate als Ausgangsprodukte zur Synthese von Phospholipiden.**

(57) Die Erfindung betrifft D-Mannitderivate der allgemeinen Formel I

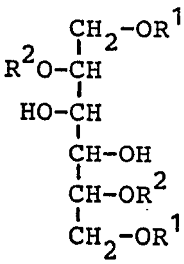

(I)

worin $R^1$ und $R^2$ gleich oder verschieden sind und, wenn $R^1$ und $R^2$ gleich sind, eine geradkettige oder verzweigte Alkyl-, Alkenyl- oder Alkinylgruppe mit 6 bis 24 Kohlenstoffatomen bedeuten, die Cycloalkylreste mit 3 bis 6 Kohlenstoffatomen, Arylreste, Benzyloxy-, Allyloxy-, Mesyloxy- und/oder Halogensubstituenten enthalten können, und, wenn $R^1$ und $R^2$ verschieden sind, eine geradkettige oder verzweigte Alkylgruppe mit 1 bis 24 Kohlenstoffatomen, die Cycloalkylreste mit 3 bis 6 Kohlenstoffatomen, Arylreste, Benzyloxy-, Allyloxy-, Mesyloxy- und/oder Halogensubstituenten enthalten können, oder eine geradkettige oder verzweigte Alkenyl- oder Alkinylgruppe mit 3 bis 24 Kohlenstoffatomen, die Cycloalkylreste mit 3 bis 6 Kohlenstoffatomen, Arylreste, Benzyloxy-, Allyloxy-, Mesyloxy- und/oder Halogensubstituenten enthalten können, bedeuten, und $R^1$ auch Trityl sein kann. Ausgehend von diesen D-Mannitderivaten lassen sich auf einfache Weise und mit guten Ausbeuten Phospholipide in Form ihrer optisch reinen Stereoisomeren erhalten.

EP 0 098 600 A2

Die Erfindung betrifft neue D-Mannitderivate als Ausgangsprodukte zur Synthese von Phospholipiden.

Die erfindungsgemäßen D-Mannitderivate eignen sich insbesondere sehr gut als Ausgangsverbindungen zur Synthese von (Diäther)-Phospholipiden, von (Monoäther)-Phospholipiden und von (Äther/Ester)-Phospholipiden und ermöglichen die Herstellung von struktur-, stereo- und positionsisomeren Verbindungen dieser Substanzklassen, deren biologische Eigenschaften neuerdings intensiv untersucht werden.

Im Vergleich zu (Diacyl)-Phospholipiden (vgl. deutsche Patentanmeldung P 31 30 867.8 vom 4. August 1981) kommen die entsprechenden Dialkyl-Glycerinderivate in tierischen Membranen allerdings kaum vor. Jedoch sind gemischte Alkyl/Acyl-Glycerinphosphatide häufig, beispielsweise die Plasmalogene, die in Organen wie Herz und Lunge wesentliche Bestandteile der Membran darstellen. Es mehren sich auch Beobachtungen, daß gewisse Äther/Ester-Phospholipide in äußerst geringen Mengen ($10^{-10}$ - $10^{-11}$ M) hormonartige Eigenschaften besitzen, wie z. B. "PAF" (Platelet Activating Factor), dessen Struktur als 1-Octadecyl-2-acetyl-sn-glycerin-3-phosphocholin beschrieben wird. Daher besteht ein großes Interesse an einfachen Synthesen, die eine spezifische Positionierung der Alkylreste oder Acylreste, unabhängig voneinander, im Glycerinmolekül erlauben.

Obwohl Diäther-Phospholipide in tierischen Membranen kaum vorkommen, sind die den Ester-Phospholipiden verwandten Strukturen von großem Interesse in der Zellbiologie. Wie in zahlreichen Untersuchungen gezeigt werden konnte (siehe dazu H. Eibl, Liposomes: From Physical

Structure to Therapeutic Application, Chapter 2: Phospholipid Syntheses; ed. C. G. Knight, Elsevier Amsterdam (1981), 19), unterscheiden sich Äther-Phospholipide von Ester-Phospholipiden kaum hinsichtlich der physikalischen Eigenschaften, aber stark in ihrer chemischen Stabilität gegenüber pH-Variationen und besonders in ihrer biologischen Stabilität gegen die Phospholipasen $A_1$ und $A_2$, die nur Esterphospholipide hydrolysieren können. Außerdem kann durch Variation des Phosphat-Trimethylammonium-Abstandes in Phosphatidylcholinen ein Abbau durch Phospholipase C und D nach Wunsch verlangsamt oder völlig unterdrückt werden (Eibl und Kovatchev, Methods of Enzymology (1981) 72, 632). Die genannten Strukturvariationen erlauben den Aufbau von Liposomen, deren biologische Stabilität nach Wunsch gesteuert, d. h. deren biologische Halbwertszeit gezielt eingestellt werden kann. Zur Darstellung dieser Liposomen werden üblicherweise Gemische von Phospholipiden eingesetzt, wie diese auch in natürlichen Membranen vorkommen (Lecithine 40 - 80 %; Kephaline 20 - 60 % und negativ geladene Phospholipide 5 - 30 %). Diäther-Phospholipide mit den genannten Strukturvariationen im polaren Bereich sind also besonders interessant zur Darstellung von Liposomen mit erhöhter biologischer Halbwertszeit.

Lysolecithinderivate besitzen wichtige biologische Wirkungen. So eignen sich Lysolecithine zum Beispiel als immunologische Adjuvantien, das sind Substanzen, die die Immunantwort des Organismus auf einen antigenen Reiz, also die Antikörperbildung, verstärken (vgl. DE-OS 20 09 343); (Äther)-Lysolecithine fördern die Steigerung der natürlichen Resistenz des Organismus (vgl. DE-OS 20 09 342). Eine große Bedeutung kommt den Lysolecithinderivaten aufgrund ihrer besonderen Wirksamkeit auf das Wachstum von Tumoren zu; sie stellen deshalb ein wirkungsvolles Anti-Tumormittel dar (vgl. DE-OS 26 19 686).

Die als Anti-Tumormittel wirksamen Lysolecithinderivate besitzen ein asymmetrisches Kohlenstoffatom und können deshalb in zwei stereoisomeren Formen (D- und L-Form) auftreten, von denen aber nur eine Form wirksam ist. Für den Einsatz als Arzneimittel sind somit die reinen stereoisomeren biologisch aktiven Formen dem Racemat überlegen. Aber auch die nicht biologisch aktiven Formen sind als reine Stereoisomere wertvolle Reagentien, insbesondere zur Ermittlung des Wirkungsmechanismus der (Äther)-Lecithine; als Racemat im Gemisch mit den biologisch aktiven Formen verabreicht, stellen sie hingegen lediglich eine Belastung des Organismus dar, also einen unerwünschten, unwirksamen Ballaststoff. Die bisher bekannten Anti-Tumormittel liegen als Racemat vor, also als Mischung der D- und L-Form.

Interessanterweise wurde neuerdings nun auch gefunden, daß zwischen normalen Zellen und Tumorzellen ein wichtiger Unterschied in der Enzymausrüstung besteht (Wykle and Snyder, The Enzymes of Biological Membranes (1976), 2, 87). In Tumorzellen fehlt ein 1-O-Alkyl-spaltendes Enzym vollkommen, während dies in der mikrosomalen Fraktion von normalen Zellen immer vorhanden ist.

Mit einigem Erfolg wurde deshalb 1-Octadecyl-2-methyl-rac-glycero-3-phosphocholin (Weltzien und Westphal (1967), 709, 240) zur Tumortherapie verwendet, da normale Zellen aufgrund ihrer 1-O-Alkyl-spaltenden Enzyme diese lytischen und toxischen Substanzen zumindest teilweise metabolisieren und entgiften, während Tumorzellen aufgrund ihrer ungenügenden Enzymausrüstung dazu nicht in der Lage sind. Leider konnten bisher optisch reine Verbindungen nicht eingesetzt werden, da entsprechende Synthesen nicht zur Verfügung standen.

Insbesondere die günstige Wirkung von 1-Octadecyl-2-methyl-rac-glycero-3-phosphocholin als neueres Anti-Tumormittel (vgl. DE-OS 20 09 342, DE-OS 20 09 343 und DE-OS 26 19 686) war Anlaß, Möglichkeiten zur Synthese der optisch reinen Verbindungen mit natürlicher Konfiguration (sn-Glycero-3-phosphocholine) zu entwickeln, um damit eine überflüssige Belastung des Organismus mit Strukturen unwirksamer Konfiguration zu vermeiden und zusätzlich eine Höherdosierung der Wirksubstanz zu ermöglichen.

Aufgabe der vorliegenden Erfindung ist es deshalb, Wege zur Synthese von Glycerinäthern, aber auch von Glycerinestern, bereitzustellen, die eine einfache Darstellung aller gewünschten Vertreter dieser Stoffklasse in den optisch reinen Formen erlauben. Diese Aufgabe wird mit der vorliegenden Erfindung gelöst.

Gegenstand der Erfindung sind D-Mannitderivate der allgemeinen Formel I (neben der Formel ist die stereospezifische Numerierung, sn, angegeben)

sn
1
2
3
4
5
6

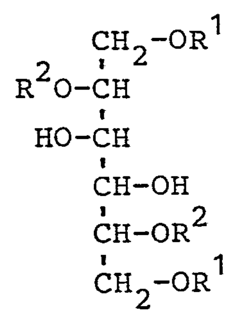

(I)

worin $R^1$ und $R^2$ gleich oder verschieden sein können und, wenn $R^1$ und $R^2$ gleich sind, eine geradkettige oder

verzweigte Alkyl-, Alkenyl- oder Alkinylgruppe mit 6 bis 24 Kohlenstoffatomen bedeuten, die Cycloalkylreste mit 3 bis 6 Kohlenstoffatomen, Arylreste, Benzyloxy-, Allyloxy-, Mesyloxy- und/oder Halogensubstituenten, wie Fluor, Brom, Jod und insbesondere Chlor, enthalten können, und, wenn $R^1$ und $R^2$ verschieden sind, eine geradkettige oder verzweigte Alkylgruppe mit 1 bis 24 Kohlenstoffatomen, die Cycloalkylreste mit 3 bis 6 Kohlenstoffatomen, Arylreste, Benzyloxy-, Allyloxy-, Mesyloxy- und/oder Halogensubstituenten enthalten können, oder eine geradkettige oder verzweigte Alkenyl- oder Alkinylgruppe mit 3 bis 24 Kohlenstoffatomen, die Cycloalkylreste mit 3 bis 6 Kohlenstoffatomen, Arylreste, Benzyloxy-, Allyloxy-, Mesyloxy- und/oder Halogensubstituenten enthalten können, bedeuten, und $R^1$ auch Trityl sein kann, und wobei vorzugsweise mindestens ein Rest $R^1$ oder $R^2$ mehr als 6 Kohlenstoffatome, und insbesondere mehr als 9 Kohlenstoffatome besitzt.

Als Substituent von $R^1$ und/oder $R^2$ bedeutet ein Cycloalkylrest mit 3 bis 6 Kohlenstoffatomen zum Beispiel Cyclopropyl, Cyclobutyl, Methyl-cyclobutyl, Cyclopentyl und insbesondere Cyclohexyl; ein Arylrest zum Beispiel Naphthyl und insbesondere Phenyl und Halogen Fluor, Brom, Jod und insbesondere Chlor.

Bevorzugte D-Mannitderivate der Formel I sind 1,6-Dibenzyl-2,5-diallyl-D-mannit, 1,6-Diallyl-2,5-dibenzyl-D-mannit, 1,6-Ditrityl-2,5-dibenzyl-D-mannit, 1,6-Ditrityl-2,5-diallyl-D-mannit, 1,6-Ditrityl-2,5-dialkyl-D-mannit, 1,6-Ditrityl-2,5-dialkenyl-D-mannit, 1,6-Ditrityl-2,5-dialkinyl-D-mannit, 1,6-Dialkyl-2,5-dibenzyl-D-mannit, 1,6-Dialkyl-2,5-diallyl-D-mannit, 1,6-Dialkyl-2,5-dialkyl-D-mannit, 1,6-Dialkenyl-2,5-dialkyl-D-mannit, 1,6-Dialkinyl-2,5-dialkyl-D-mannit, 1,6-Dialkyl-2,5-dialkenyl-D-mannit, 1,6-Dialkenyl-2,5-dial-

kenyl-D-mannit, 1,6-Dialkinyl-2,5-dialkenyl-D-mannit, 1,6-Dialkyl-2,5-dialkinyl-D-mannit, 1,6-Dialkenyl-2,5-dialkinyl-D-mannit, 1,6-Dialkinyl-2,5-dialkinyl-D-mannit, insbes. 1,6-Dioctadecyl-2,5-dimethyl-D-mannit, 1,6-Dimethyl-2,5-dioctadecyl-D-mannit, 1,6-Dioctadecyl-2,5-diallyl-D-mannit, 1,6-Dioctadecyl-2,5-dibenzyl-D-mannit, 1,6-Dioctadec-(3')-enyl-2,5-dimethyl-D-mannit, 1,6-Dioctadec-(3')-inyl-2,5-dimethyl-D-mannit, 1,6-Dioctadecyl-2,5-ditetradecyl-D-mannit, 1,6-Dioctadecyl-2,5-didodecyl-D-mannit, 1,6-Dioctadecyl-2,5-didecyl-D-mannit, 1,6-Dioctadecyl-2,5-dioctyl-D-mannit, 1,6-Dioctadecyl-2,5-dibutyl-D-mannit, 1,2,5,6-Tetraoctadecyl-D-mannit, 1,2,5,6-Tetrahexadecyl-D-mannit, 1,2,5,6-Tetra-(tetradecyl)-D-mannit, 1,6-Ditrityl-2,5-dioctadecyl-D-mannit, 1,6-Ditrityl-2,5-dihexadecyl-D-mannit, 1,6-Ditrityl-2,5-ditetradecyl-D-mannit, 1,6-Ditrityl-2,5-didodecyl-D-mannit, 1,6-Ditrityl-2,5-didecyl-D-mannit, 1,6-Ditrityl-2,5-dioctyl-D-mannit und 1,6-Ditrityl-2,5-dihexyl-D-mannit.

Gegenstand der Erfindung ist auch ein Verfahren zur Herstellung der D-Mannitderivate der allgemeinen Formel I, das dadurch gekennzeichnet ist, daß man 3,4-Isopropyliden-D-mannit zum 1,6-Ditrityl-3,4-isopropyliden-D-mannit trityliert, in die so erhaltene Verbindung die Gruppe $R^2$ durch Umsetzung mit dem entsprechenden $R^2$-Mesylat, -Chlorid, -Bromid oder -Jodid oder mit $(R^2O)_2SO_2$ einführt, danach für den Fall, daß $R^1$ nicht Trityl ist, die Tritylgruppe in schwach saurem Medium abspaltet, und in das enttritylierte Produkt die Gruppe $R^1$ durch Umsetzung mit dem entsprechenden $R^1$-Mesylat, -Chlorid, -Bromid oder -Jodid oder mit $(R^1O)_2SO_2$ einführt, und dann die Isopropylidengruppe abspaltet.

Ausgehend von den erfindungsgemäßen D-Mannitderivaten

der Formel I ist sowohl die Darstellung der Formen mit natürlicher Konfiguration (z. B. 1-Octadecyl-2-methyl-sn-glycero-3-phosphocholin und 1-Methyl-2-octadecyl-sn-glycero-3-phosphocholin), als auch der Formen mit nicht natürlicher Konfiguration (z. B. 3-Octadecyl-2-methyl-sn-glycero-1-phosphocholin, 3-Methyl-2-octadecyl-sn-glycero-1-phosphocholin, 1-Octadecyl-3-methyl-sn-glycero-2-phosphocholin und 1-Methyl-3-octadecyl-sn-glycero-2-phosphocholin auf einfachem Wege möglich. Entsprechend wurden Phospholipide mit negativer und positiver Oberflächenladung dargestellt, die Octadecylmethyl-glycerine als apolare Grundstrukturen enthalten. Besonders solche polaren Strukturen wurden ausgewählt, die die Natur vorbildlich zum Aufbau von Membranen verwendet. Neben den im physiologischen Milieu neutralen Phosphocholinen und Phosphoäthanolaminen wurden erstmals auch neue Phosphoglycerine, Phosphoserine und Phosphatidsäuren (Äther-Phospholipide) dargestellt, die sich durch eine negative Oberflächenladung bei pH 7 auszeichnen. Entsprechende Strukturen mit positiver Überschußladung wurden ebenfalls dargestellt. Die neuen reinen stereoisomeren Äther-Phospholipide sind im Hinblick auf die tumorwachstumshemmenden Eigenschaften von besonderem Interesse, da

1. mit den optisch reinen Vertretern eine höhere Dosierung möglich wird;

2. eine zusätzliche Belastung des Organismus mit Strukturen unwirksamer Konfiguration nicht erfolgt;

3. über entsprechende Ladungsmuster (positiv, negativ, neutral) in Dialkyl-Phospholipiden bestimmte Tumoren möglicherweise selektiv angesteuert werden können;

4. In konsequenter Ausnützung der Beobachtung, daß Tumorzellen keine 1-O-Alkylspaltenden Enzyme besitzen, können nach den hier vorgeschlagenen Methoden Phospholipide vom Äther/Ester-Typ konstruiert werden, die Doppelschichten bilden und damit Membranstabilisierend wirken, z. B. 1-Octadecyl-2-oleoyl-sn-glycero-3-phospho-N,N,N-(trimethyl)-hexanolamin. Dieses Phospholipid ist untoxisch, aber ein Substrat für die ubiquitären Phospholipasen $A_2$. Es wird deshalb sowohl in normalen Zellen als auch in Tumorzellen durch Abspaltung von Ölsäure in das toxische 1-Octadecyl-sn-glycero-3-phospho-N,N,N-(trimethyl)-hexanolamin übergehen und kann aufgrund des vergrößerten Phosphat-trimethylammoniumabstandes nicht reacyliert und entgiftet werden. Nur den normalen Zellen verbleibt die Abwehrmöglichkeit über die dort vorhandenen 1-O-Alkylspaltenden Enzyme. Die Tumorzellen sterben durch Anreicherung von 1-Octadecyl-sn-glycero-3-phospho-N,N,N-(trimethyl)-hexanolamin ab.

Die Mannitderivate der Formel I können quantitativ durch Diolspaltung und Reduktion in Glycerinderivate der nachstehenden Formel II umgewandelt werden. Enthält Formel II einen Benzylrest in der sn-1-Position, so kann durch Alkylierung in 3-Position und katalytische Debenzylierung eine Konfigurationsumkehr zu Formel III erreicht werden. Entsprechend entsteht Formel IV unter Konfigurationsänderung, wenn der Benzylrest in der sn-2-Position des Glyceringrundgerüstes von Formel II verankert ist. Statt Benzyl kann auch Allyl, in 1- oder 3-Position auch Trityl verwendet werden.

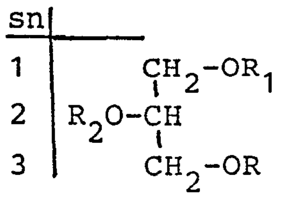 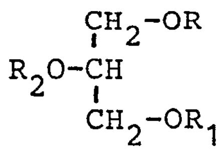 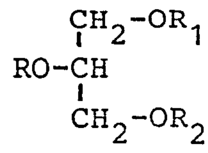

II III IV

Aus den Glycerinderivaten der Formeln II, III und IV (R = H) können durch geeignete Phosphorylierungsschritte, meist über die nicht isolierten Phosphorsäuredichloride V, VI und VII , die entsprechenden Phospholipide der Formel VIII, IX und X erhalten werden (VIII, natürlich, sn-3-Phosphat; IX, nicht natürlich, sn-1-Phosphat; X, nicht natürlich, sn-2-Phosphat):

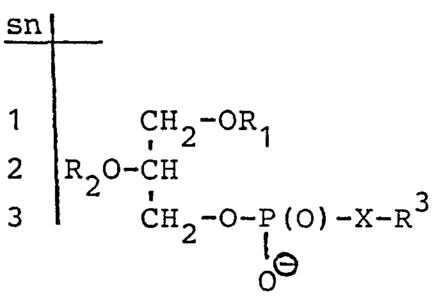 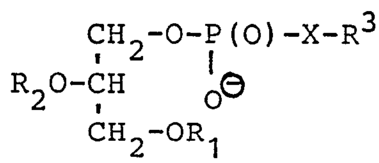

VIII IX

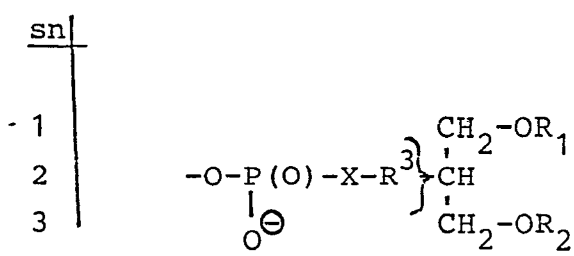

X

Die Synthese von Ester-Phospholipiden oder von Äther/Ester-Phospholipiden kann, ausgehend von den entsprechend substituierten Glycerinderivaten der Formeln II bis IV nach einem der in der deutschen Patentanmeldung P 31 30 867.8 vom 4. August 1981 beschriebenen Verfahren erfolgen.

Gegenstand der Erfindung sind deshalb auch Glycerinderivate der allgemeinen Formeln II, III und IV

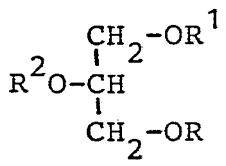

II

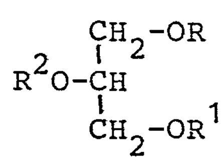

III

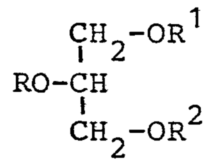

IV

worin $R^1$ und $R^2$ die oben für die allgemeine Formel I angegebenen Bedeutungen besitzen und R ein Wasserstoffatom oder den Rest

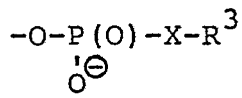

darstellt, worin X = 0, NH oder $NR^3$ ist, und, wenn X = 0 ist, $R^3$ H, Alkyl, Alkenyl oder Alkinyl mit 1 bis 18 Kohlenstoffatomen, Halogenalkyl mit 2 bis 14 Kohlenstoffatomen, 2-Amino-2-carboxy-äthyl (Serinrest), Dihydroxypropyl (Glycerinrest), Pentahydroxyhexyl (D-Mannitrest), Aminoalkyl mit 2 bis 14 Kohlenstoffatomen, N-Methyl-aminoalkyl mit 2 bis 14 Kohlenstoffatomen, N,N-Dimethyl-aminoalkyl mit 2 bis 14 Kohlenstoffatomen, N,N,N-Trimethyl-aminoalkyl mit 2 bis 14 Kohlenstoffatomen, N-/(N,'N,'N'-Trimethyl)-aminoäthyl7-N,N-dimethyl-aminoäthyl bedeutet, wenn X = NH ist, $R^3$ H, Alkyl oder Alkenyl mit 1 bis 10 Kohlenstoffatomen, Halogenalkyl mit 2 bis 6 Kohlenstoffatomen, Hydroxyäthyl, Dihydroxypropyl, Aminoalkyl bedeutet, und, wenn X = $NR^3$ ist, $R^3$ Alkyl, Alkenyl mit 1 bis 10 Kohlenstoffatomen, Bis-Chloräthyl bedeutet, und wobei, wenn R kein Wasserstoffatom ist, einer der Reste $R^1$ oder $R^2$ in Formel II, III oder IV auch ein Wasserstoffatom sein kann.

Weiterer Gegenstand der Erfindung ist auch ein Verfahren zur Weiterverarbeitung der D-Mannitderivate der Formel I zur Herstellung der Glycerinderivate der Formeln II, III oder IV, das dadurch gekennzeichnet ist, daß man

a) zur Herstellung der Verbindungen der Formel II die D-Mannitderivate der Formel I mit Bleitetraacetat umsetzt und in dem entstandenen Glycerinaldehydderivat die CHO-Gruppe zur $CH_2OH$-Gruppe reduziert,

b) zur Herstellung der Verbindungen der Formel III von D-Mannitderivaten der Formel I ausgeht, in denen $R^1$ eine Tritylgruppe, oder, wenn $R^1$ in Formel III gesättigt ist, auch eine Benzylgruppe ist, mit Bleitetraacetat umsetzt, in dem entstandenen Glycerinaldehydderivat die CHO-Gruppe zur $CH_2OH$-Gruppe reduziert, in die so erhaltene Verbindung die Gruppe $R^1$ durch Umsetzung mit dem entsprechenden $R^1$-Mesylat, -Chlorid, -Bromid oder -Jodid oder mit $(R^1O)_2SO_2$ einführt und danach die Tritylgruppe durch saure Hydrolyse oder die Benzylgruppe durch katalytische Hydrogenolyse abspaltet,

c) zur Herstellung von Verbindungen der Formel IV von D-Mannitderivaten der Formel I ausgeht, in denen $R^1$ eine Alkylgruppe und $R^2$ eine Allylgruppe oder, wenn $R^1$ und $R^2$ in Formel IV gesättigt sind, auch eine Benzylgruppe ist, mit Bleitetraacetat umsetzt, in dem entstandenen Glycerinaldehydderivat die CHO-Gruppe zur $CH_2OH$-Gruppe reduziert, in der so erhaltenen Verbindung gegebenenfalls die Allylgruppe in eine Propenylgruppe umlagert, $R^2$ durch Umsetzung mit dem entsprechenden $R^2$-Mesylat, -Chlorid, -Bromid oder -Jodid oder mit $(R^2O)_2SO_2$ einführt und danach die Propenylgruppe durch saure Hydrolyse oder die Benzylgruppe

durch katalytische Hydrogenolyse wieder abspaltet, und wenn erwünscht, in die nach a), b) oder c) erhaltenen Verbindungen, in denen R ein Wasserstoffatom bedeutet, den Rest

$$\begin{array}{l} -O-P(O)-X-R^3 \\ \;\;| \\ \;\;O^{\ominus} \end{array}$$

auf an sich bekannte Weise einführt, und, wenn erwünscht, in einer Verbindung der Formel II, III oder IV, in der R kein Wasserstoffatom bedeutet, einen Benzyl- oder Allylrest $R^1$ oder $R^2$ auf an sich bekannte Weise in ein Wasserstoffatom überführt.

Wenn erwünscht oder zweckmäßig, können die Verfahren auch so durchgeführt werden, daß man von einem nach einer der Verfahrensstufen erhaltenen Produkt ausgeht und die restlichen Verfahrensstufen durchführt.

Die Tritylierung (Einführung der Tritylgruppe) erfolgt vorzugsweise durch Umsetzung mit Tritylchlorid in Gegenwart eines tertiären Amins, z. B. Triäthylamin, in einem geeigneten organischen Lösungsmittel. Für diese Umsetzung geeignete Lösungsmittel sind z. B. polare organische Lösungsmittel, wie zum Beispiel sekundäre oder tertiäre Alkanole, zum Beispiel tert.-Butanol, Dioxan, Tetrahydrofuran, aber auch Benzol. Die Reaktion wird normalerweise bei Siedetemperatur des Lösungsmittels durchgeführt. Die Beendigung der Reaktion kann durch Dünnschichtchromatographie festgestellt werden. Vor der Weiterverarbeitung (z. B. Einführung des Restes $R^2$) ist es zweckmäßig, das erhaltene Produkt einer Reinigungsstufe zu unterziehen. Dazu wird zum Beispiel die Hauptmenge Lösungsmittel, zum Beispiel tert.-Butanol, im Vakuum abgedampft, der Rückstand in Diisopropyläther aufgenommen, die Ätherphase zweimal mit Wasser gewaschen und dann eingedampft.

Die Umsetzung mit Alkyl-mesylat, -chlorid, -bromid oder -jodid oder mit Dialkylsulfat kann unter den für eine solche Umsetzung üblichen bekannten Bedingungen erfolgen. Als Lösungsmittel können die für derartige Umsetzungen üblichen Lösungsmittel, wie zum Beispiel tertiäre Alkohole, Tetrahydrofuran, Xylol oder Dioxan, eingesetzt werden. Die Reaktionstemperaturen liegen normalerweise über Raumtemperatur, vorzugsweise zwischen 50 und 100°C, zum Beispiel bei Siedetemperatur des Lösungsmittels. Als Base werden Alkylate verwendet, insbesondere das Kalium-tert.-butylat. Zur Einführung

langkettiger Alkylreste ist es zweckmäßig, anstelle der Halogenide die entsprechenden Mesylate zu verwenden. Die Beendigung der Reaktion kann durch Dünnschichtchromatographie festgestellt werden. Vor der Weiterverarbeitung ist es zweckmäßig, das erhaltene Produkt einer Reinigungsstufe zu unterziehen. Dazu wird das Reaktionsgemisch zum Beispiel mit Diisopropyläther versetzt und mit Wasser extrahiert. Die Ätherphase wird eingedampft und kann chromatographisch, zum Beispiel an Kieselgel, gereinigt werden.

Die Abspaltung der Tritylgruppe erfolgt unter schwach sauren Bedingungen, vorzugsweise bei einem pH-Wert von 4 bis 6, wobei der günstigste Wert unter Berücksichtigung der übrigen Substituenten im Molekül leicht eruiert werden kann. Die Allyl- und Benzylschutzgruppen sind hierbei vollkommen stabil. Die Reaktion kann in einem wäßrigen oder wäßrig-organischen, aber auch in einem rein organischen Medium, wie zum Beispiel in absolutem Äthanol, in Gegenwart von HCl oder $H_2SO_4$ durchgeführt werden. Das organische Lösungsmittel kann dabei ein mit Wasser mischbares oder aber auch ein mit Wasser nur teilweise oder wenig mischbares inertes Lösungsmittel sein. Die Reaktion erfolgt, insbesondere beim Arbeiten in einem Zweiphasensystem, vorteilhafterweise unter starkem Rühren. Die Temperatur beträgt im allgemeinen 20 bis 80°C. Zur Verbesserung der Löslichkeit kann es zweckmäßig sein, einen höheren Alkohol, wie zum Beispiel Propanol-(2), in kleiner Menge zuzusetzen. Beispielsweise wird die tritylierte Verbindung in Aceton gelöst, dann die gleiche Menge konzentrierte Schwefelsäure enthaltendes Methanol (Methanol/Schwefelsäure = 1/100) zugefügt und bei 50°C ca. 60 Minuten erwärmt. Zur Reinigung wird das erhaltene Reaktionsgemisch zum Beispiel mit Diisopropyläther versetzt, zweimal mit Wasser und danach mit wäßrigem 1 M-Natriumbicarbonat gewaschen. Die Ätherphase wird eingedampft und kann chromatographisch, zum Beispiel an Kieselgel, gereinigt werden.

Die Abspaltung der Isopropylidengruppe kann unter sauren Bedingungen in einem organisch-wäßrigen Medium erfolgen. So wird zum Beispiel das nach der Abspaltung der Tritylgruppe und Einführung des Restes $R^1$ erhaltene Reaktionsprodukt in einem Gemisch aus Propanol-(2)/Methanol (1/1) mit 2 N Schwefelsäure (50 ml auf 1 l Lösungsmittel) versetzt und unter Rückfluß gekocht. Das Ende der Reaktion kann durch DC-Kontrolle festgestellt werden.

Die Spaltung des Mannitgrundgerüstes in zwei Glycerinmoleküle erfolgt durch Reaktion mit Bleitetraacetat in einem organischen, gegenüber Bleitetraacetat inerten Lösungsmittel, wie zum Beispiel in Benzol, Toluol, Tetrahydrofuran oder Dioxan. Beispielsweise wird das Bleitetraacetat zur Lösung bei Raumtemperatur portionsweise und unter Rühren zugegeben; nach Beendigung der Reaktion (Verschwinden des Ausgangsproduktes, DC-Kontrolle) wird dann die Reaktionslösung zweimal mit Wasser gewaschen und das Lösungsmittel (zum Beispiel Benzol) abgedampft.

Die Reduktion der CHO-Gruppe zur $CH_2OH$-Gruppe kann auf an sich bekannte Weise durch Reduktion mit einem Metallhydrid in einem geeigneten organischen Lösungsmittel erfolgen, zum Beispiel mit Alkalimetallaluminiumhydrid, wie zum Beispiel Lithiumaluminiumhydrid, oder mit Alkalimetallborhydrid, wie zum Beispiel mit Kaliumborhydrid, und insbesondere mit Natriumborhydrid. Vorzugsweise erfolgt die Reduktion mit Natriumborhydrid in Methanol als Lösungsmittel.

Die Einführung der Benzylgruppe kann auf an sich bekannte Weise erfolgen, wie zum Beispiel durch Umsetzung mit Benzylchlorid unter den oben für die Umsetzung mit Alkylhalogeniden angegebenen Bedingungen.

Die Abspaltung der Benzylgruppe erfolgt durch katalytische Hydrogenolyse. Die Reaktionsbedingungen entsprechen dabei den üblichen Bedingungen. Insbesondere führt man die Hydrogenolyse in einem inerten Lösungsmittel, wie zum Beispiel Äthanol, in Gegenwart eines Palladium- oder Platin/Palladium-Katalysators durch, vorzugsweise bei Raumtemperatur und unter Normaldruck (vgl. H. Eibl et al, Liebigs Annalen Chemie, 738, (1970), 161).

Die Abspaltung der Allylgruppe (Umlagerung in Propenyl und nachfolgende Abspaltung von Propenyl) kann nach zwei verschiedenen Methoden erfolgen, nämlich 1) unter alkalischen Bedingungen, wie zum Beispiel mit Kalium-tert.-butylat in Dimethylformamid und anschließende Spaltung mit Brom in gepufferter Lösung bei einem pH-Wert um 5 bis 6, oder 2) durch Umlagerung in Gegenwart eines Palladium-(Kohle)-Katalysators unter Bildung der unter diesen Bedingungen instabilen, spontan abspaltenden Propenylgruppe, wobei zweckmäßigerweise in 80 %-igem Methanol, welches in der wäßrigen Phase 20 % Ameisensäure enthält, bei Rückflußtemperatur gearbeitet wird. Im allgemeinen ist die Variante 1, d. h. die Abspaltung mit Brom, bevorzugt. Zur Abspaltung der Propenylgruppe in 1-Stellung kann auch Jod verwendet werden (Eibl und Lands, Biochemistry 9 (1970), 423). Während aber eine Abspaltung der Propenylgruppe in 2-Stellung mit Jod überhaupt nicht möglich ist, läßt sich eine solche Abspaltung überraschenderweise mit Brom vollständig und in wenigen Minuten durchführen.

Die Einführung des Restes

$$-O-\underset{O^{\ominus}}{P}(O)-X-R^3$$

erfolgt auf an sich bekannte Weise, wie zum Beispiel durch Umsetzung mit Halogen(vorzugsweise Brom)-alkylphosphorsäuredichlorid (vgl. Hirth und Berchthold, Pharm. Acta. Helv. 33 (1958), 349) oder vorzugsweise durch Umsetzung mit $POCl_3$ in Gegenwart eines tertiären Amins, insbesondere von Triäthylamin, und nachfolgende Umsetzung des erhaltenen Glycerin-phosphorsäure-dichlorids mit dem entsprechenden Halogen(vorzugsweise Brom)-alkanol (H. Eibl, Proc. Nat. Acad. Sci. USA 75 (1978), 4074; H. Eibl und A. Nicksch, Chem. Phys. Lipids 22 (1978), 1; W. Diembeck und H. Eibl, Chem. Phys. Lipids 24 (1979), 237), und ganz bevorzugt durch Umsetzung mit $POCl_3$ in Gegenwart eines tertiären Amins, nachfolgende Umsetzung mit dem entsprechenden Bromalkanol in Gegenwart eines tertiären Amins und anschließende Methanolyse, an die sich dann zum Beispiel die Umsetzung mit dem entsprechenden Amin anschließt. Das Glycerinphosphorsäuredichlorid kann aber auch mit dem entsprechenden Aminoalkohol umgesetzt und die Aminogruppe z. B. nachträglich alkyliert werden.

Die Umsetzung mit dem Amin erfolgt in an sich bekannter Weise (vgl. H. Eibl und A. Nicksch, Chem. Phys. Lipids 22 (1978), 1; W. Diembeck und H. Eibl, Chem. Phys. Lipids 24 (1979), 237), ebenso die nachträgliche Alkylierung der freien Aminogruppe. Als Lösungsmittel für die Umsetzungen mit Phosphoroxychlorid und Halogenalkanol kann zum Beispiel Dioxan, Chloroform, Tetrachlorkohlenstoff, Methylenchlorid und insbesondere Tetrahydrofuran dienen. Zweckmäßigerweise wird zum Beispiel

das nach der Verfahrensstufe der Reduktion mit Natriumborhydrid erhaltene Produkt (0,1 Mol) in 500 ml Tetrahydrofuran gelöst und mit 0,2 Mol Triäthylamin versetzt. Diese Lösung wird bei 20°C zu Phosphoroxychlorid (0,15 Mol) in 100 ml Tetrahydrofuran getropft. Nach Beendigung der Reaktion (DC-Kontrolle) wird von ausgefallenem Triäthylaminhydrochlorid abfiltriert, mit Toluol versetzt und zur Entfernung von überschüssigem $POCl_3$ einrotiert. Man nimmt den Rückstand in 400 ml Tetrahydrofuran auf und versetzt mit 0,2 Mol Triäthylamin. Bei 20 bis 30°C werden 0,2 Mol Bromäthanol in 200 ml Tetrahydrofuran eingetropft. Nach Beendigung der Reaktion (DC-Kontrolle) wird von Triäthylaminhydrochlorid abfiltriert und zur Hydrolyse der PCl-Verbindungen mit 80 ml Wasser versetzt. Nach Beendigung der Reaktion (DC-Kontrolle) wird mit 600 ml Chloroform versetzt und gegen 600 ml Wasser ausgeschüttelt. Die Chloroformphase enthält das Reaktionsprodukt, das dann mit Trimethylamin umgesetzt wird, vorzugsweise in Chloroform/Propanol-(2) (1/1) als Lösungsmittel. Dazu wird zum Beispiel zu der in der vorhergehenden Stufe erhaltenen Lösung des Reaktionsproduktes in Chloroform eine 30 %-ige Lösung von Trimethylamin in Propanol-(2) zugegeben, bis ein Verhältnis Chloroform/Propanol-(2) von 1/1 erhalten ist.

Nach dem von den D-Mannitderivaten der allgemeinen Formel I ausgehenden erfindungsgemäßen Verfahren ist es möglich, Phospholipide auf einfache Weise und mit guten Ausbeuten in Form ihrer optisch reinen D- oder L-Stereoisomeren zu erhalten. Die bekannten Phospholipidsynthesen gehen von 1,2-Isopropyliden-sn-glycerin aus, dessen Synthese mit Schwierigkeiten verbunden ist (vgl. zum Beispiel H. Eibl, Chem. Phys. Lipids 1981, 28, 1 - 5). So betragen die Ausbeuten

an 1,2-Isopropyliden-sn-glycerin aus D-Mannit nur ca. 40 %, und außerdem kann ohne aufwendige Gegenmaßnahmen beim Stehen dieser Zwischenverbindung an der Luft durch die Einwirkung von Kohlendioxid Racemisierung eintreten, wodurch eine stereospezifische Synthese ohne vorherige arbeitsintensive Racematspaltung nicht möglich ist. Mit den erfindungsgemäßen Verfahren können diese Schwierigkeiten überwunden werden. So wird zum Beispiel das Ausgangsprodukt zur Herstellung der D-Mannitderivate der Formel I (3,4-Isopropyliden-D-mannit) aus D-Mannit in über 90 %-iger Ausbeute erhalten (L. F. Wiggins, J. Chem. Soc. 13 (1946)).

Die D-Mannitderivate der Formel I sind demnach wertvolle Zwischenprodukte zur stereospezifischen Synthese von Glycerinderivaten, insbesondere von Phospholipiden, der allgemeinen Formeln II bis IV.

Durch positionsspezifische Synthesen können dabei mit völliger Konfigurationserhaltung die Reste $R^1$, $R^2$ und der Phosphor enthaltende Rest nach Wunsch positioniert werden, wobei die spezifische Positionierung bereits im Mannitgrundgerüst erfolgt. Dies wird zum Beispiel in den nachfolgenden Reaktionsschemen A bis F für einige bevorzugte Verbindungen gezeigt. Überraschenderweise ist es dabei möglich, aus den Zwischenprodukten der Formel I trotz nachfolgender Umsetzung mit Bleitetraacetat und Reduktion mit guten Ausbeuten die reinen stereoisomeren Formen zu erhalten.

Im Vergleich zu früheren Untersuchungen mit racemischen Verbindungen, wie z. B. dem 1-Octadecyl-2-methylglycero-3-phosphocholin, werden erstmals die optisch reinen Enantiomeren beschrieben. Wie bereits erwähnt, eröffnet die Synthese von Ätherphospholipiden mit negativer oder positiver Überschußladung außerdem neue Möglichkeiten zur selektiven Anreicherung der tumorwachstumshemmenden Substanzen in Geweben. Die neuen Substanzen mit negativer oder positiver Überschußladung sind den human vorkommenden Phospholipiden sehr ähnlich, zum Unterschied zu den bisher verwendeten Racematen. Das gilt insbesondere für die Verbindungen plasmalogenartiger Struktur (Reaktionsschema D und E).

Gegenstand der Erfindung sind auch Arzneimittel, die eine oder mehrere der erfindungsgemäßen Verbindungen der Formel II bis IV und insbesondere II, in denen R den Rest

$$-O-\underset{O^-}{\underset{|}{P}}(O)-X-R^3$$

bedeutet, als Wirkstoff enthalten. Neben den üblichen pharmazeutischen Konfektionierungs- und/oder Verdünnungsmitteln können diese Arzneimittel neben den Verbindungen der Formeln II bis IV zur Unterstützung der Therapie gegebenenfalls auch noch weitere Wirkstoffe enthalten, sofern diese mit den erfindungsgemäßen Verbindungen der Formeln II bis IV zusammen keine unerwünschten Nebenwirkungen zeigen.

Die Wirksamkeit von Verbindungen der allgemeinen Formel I auf das Wachstum von Tumoren wird zweckmäßig an Tumoren in Versuchstieren bewiesen. Hierfür kommen verschiedene experimentelle Tumoren zur Verwendung, beispielsweise der Ehrlich-Ascites-Tumor, ein Methylcholanthreninduzierter Tumor und ein Myelom-Tumor in Mäusen, ferner ein chemisch induzierter Rattentumor. Die Anti-Tumorsubstanzen werden parenteral in die tumortragenden Versuchstiere verabreicht. Bevorzugt wird die intravenöse und die intra- bzw. subkutane Applikation. Auch die

orale Applizierbarkeit ist bei entsprechend höherer Dosierung des Anti-Tumormittels und bei einer physiologisch verträglichen Zubereitung, z. B. in Kapseln, nicht ausgeschlossen.

Als Dosierung hat sich bei der parenteralen Applikation zweckmäßig erwiesen, etwa 0,05 bis 5 mg/kg Körpergewicht einzusetzen. Um die Anti-Tumormittel über längere Zeit im Kreislauf persistieren zu lassen, ist es häufig sinnvoll, die Mittel täglich oder in 2- bis 3-tägigen Abständen zu applizieren.

In den Reaktionsschemen A bis F sind die Verfahren zur Synthese der erfindungsgemäßen Verbindungen zusammengestellt. In den Reaktionsschemen A bis C wird gezeigt, wie aus den D-Mannitderivaten der Formel I die drei stereoisomeren Glycerinabkömmlinge der Formeln II bis IV erhalten werden können. Von jeder stereoisomeren Form können für ein Dialkylätherpaar noch zwei positionsisomere Formen erhalten werden; damit können alle sechs möglichen isomeren Formen aus einem Zwischenprodukt durch einfache Syntheseschritte erhalten werden. In den Reaktionsschemen D und E finden sich dann Angaben, wie ungesättigte Reste positioniert werden können, gezeigt am Beispiel der Positionierung ungesättigter Reste in sn-1-Position und sn-2-Position des Glycerinmoleküls. Entsprechend können nach Schema A bis C auch ungesättigte Reste in sn-3-Position eingeführt werden. In Reaktionsschema F sind dann abschließend die Möglichkeiten zur Einführung des Phosphatrestes beschrieben. In den Reaktionsschemen bedeutet Jp den Isopropylidenrest.

A) 1-Octadecyl-2-methyl-sn-glycerin und 1-Methyl-2-octadecyl-sn-glycerin (durch Austausch Methylierung gegen Octadecylierung und umgekehrt).

Konfiguration: natürlich <u>(sn-3-OH)</u>

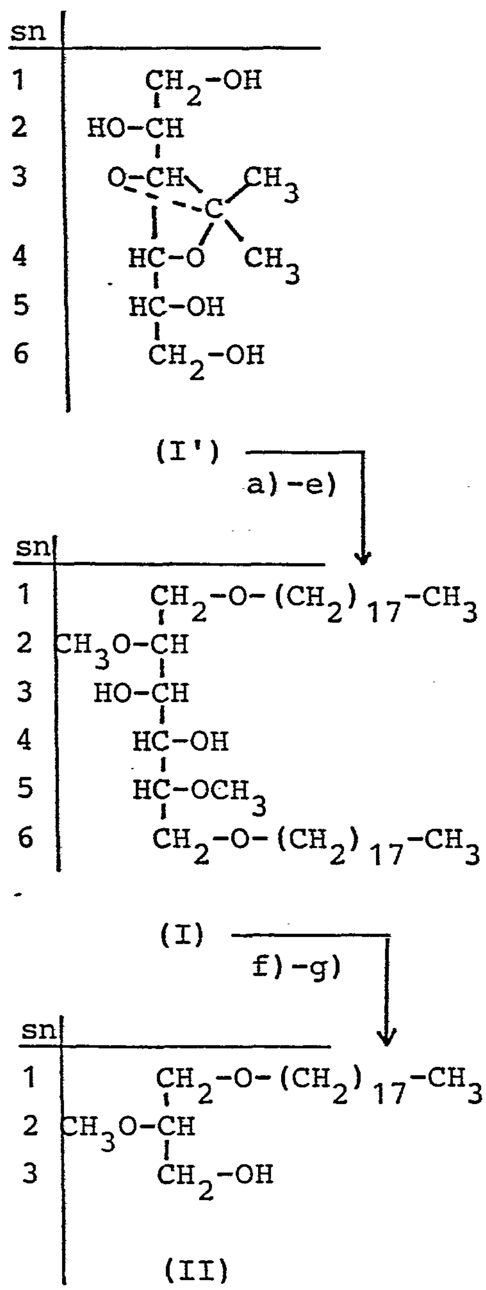


(I')

(I)

(II)

I': a) Tritylierung ⟶ 1,6-Ditrityl-3,4-Jp-D-mannit

b) Alkylierung ⟶ 1,6-Ditrityl-2,5-dimethyl-3,4-Jp-D-mannit

c) Detritylierung ⟶ 2,5-Dimethyl-3,4-Jp-D-mannit

d) Alkylierung ⟶ 1,6-Dioctadecyl-2,5-dimethyl-3,4-Jp-D-mannit

e) Deacetonierung ⟶ I; 1,6-Dioctadecyl-2,5-dimethyl-D-mannit

I: f) Diolspaltung ⟶ 1-Octadecyl-2-methyl-sn-glycerin-3-aldehyd

g) Reduktion ⟶ II; 1-Octadecyl-2-methyl-sn-glycerin

B) 3-Octadecyl-2-methyl-sn-glycerin (siehe Schema, über Konfigurationsumkehr durch spezifische Alkylierung von 1-Benzyl-2-methyl-sn-glycerin) und 3-Methyl-2-octadecyl-sn-glycerin (durch Austausch Methylierung gegen Octadecylierung und umgekehrt). Konfiguration: nicht natürlich <u>(sn-1-OH)</u>

(I')

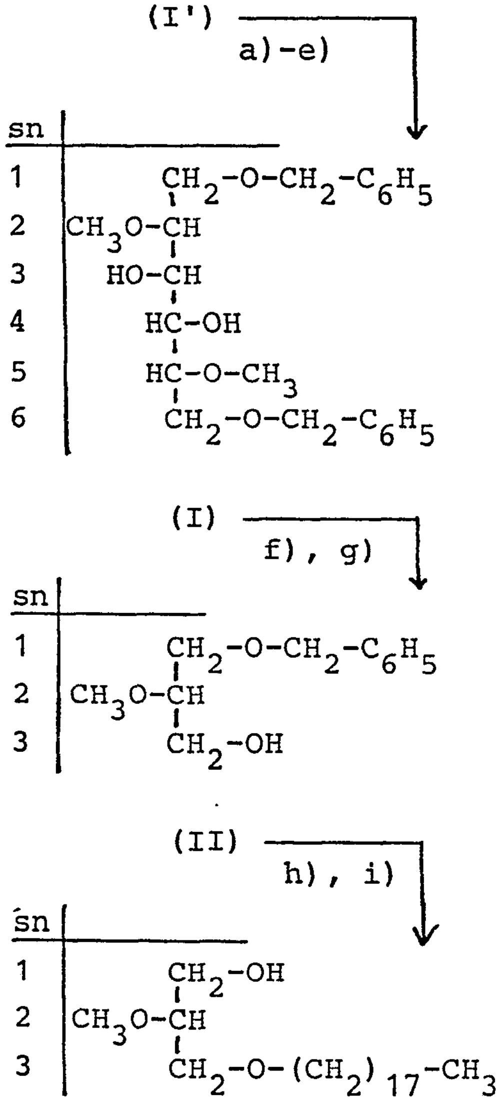


(I)

(II)

(III)

I': a) Tritylierung → 1,6-Di-trityl-3,4-Jp-D-mannit

b) Alkylierung → 1,6-Di-trityl-2,5-dimethyl-3,4-Jp-D-mannit

c) Detritylierung →2,5-Dimethyl-3,4-Jp-D-mannit

d) Alkylierung → 1,6-Di-benzyl-2,5-dimethyl-3,4-Jp-D-mannit

e) Deacetonierung: → I; 1,6-Dibenzyl-2,5-di-methyl-D-mannit

I: f) Diolspaltung von IV → 1-Benzyl-2-methyl-sn-glycerin-3-aldehyd

g) Reduktion → II; 1-Benzyl-2-methyl-sn-glycerin

<u>Konfigurationsumkehr:</u>

II: h) Alkylierung → 1-Benzyl-2-methyl-3-octadecyl-sn-glycerin

i) Kat. Debenzylierung → III; 3-Octadecyl-2-methyl-sn-glycerin

C) 1-Methyl-3-octadecyl-sn-glycerin (über Konfigurationsänderung durch spezifische Alkylierung von 1-Methyl-2-benzyl-sn-glycerin) und 1-Octadecyl-2-methyl-sn-glycerin (durch Austausch Methylierung gegen Octadecylierung und umgekehrt). Konfiguration: nicht natürlich (sn-2-OH)

(I')

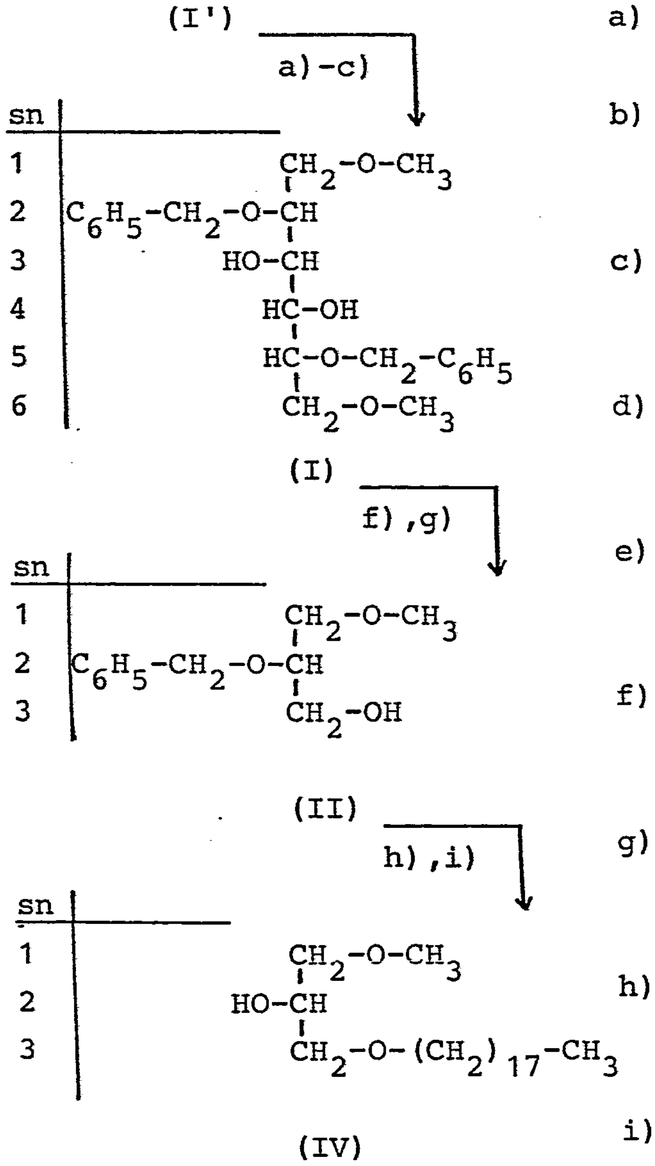


(I)

(II)

(IV)

a) Tritylierung → 1,6-Di-trityl-3,4-Jp-D-mannit

b) Alkylierung → 1,6-Di-trityl-2,5-dibenzyl-3,4-Jp-D-mannit

c) Detritylierung → 2,5-Dibenzyl-3,4-Jp-D-mannit

d) Alkylierung → 1,6-Di-methyl-2,5-dibenzyl-3,4-Jp-D-mannit

e) Deacetonierung → I; 1,6-Dimethyl-3,4-Jp-D-mannit

f) Diolspaltung → 1-Methyl-2-benzyl-sn-glycerin-3-aldehyd

g) Reduktion → II; 1-Methyl-2-benzyl-sn-glycerin

h) Alkylierung → 1-Methyl-2-benzyl-3-octadecyl-sn-glycerin

i) Kat. Debenzylierung → IV; 1-Octadecyl-2-methyl-sn-glycerin

D) Einführung von Alkenylresten in die (sn-2-)-Position des Glycerins (natürliche Konfiguration)

(I')

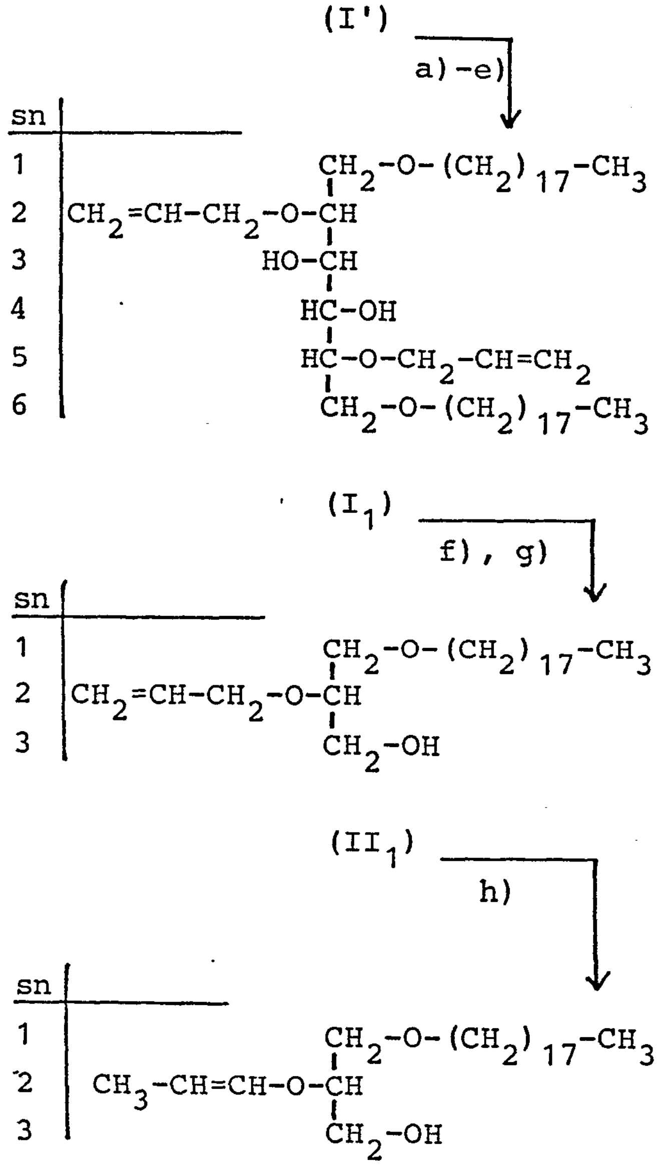


($I_1$)

($II_1$)

($II_2$)

I':a) Tritylierung → 1,6-Ditrityl-3,4-Jp-D-mannit

b) Alkylierung: → 1,6-Ditrityl-2,5-diallyl-3,4-Jp-D-mannit

c) Detritylierung: → 2,5-Diallyl-3,4-Jp-D-mannit

d) Alkylierung: → 1,6-Dioctadecyl-2,5-diallyl-3,4-Jp-D-mannit

e) Deacetonierung: → $I_1$; 1,6-Dioctadecyl-2,5-diallyl-D-mannit

$I_1$:f) Diolspaltung → 1-Octadecyl-2-allyl-sn-glycerin-3-aldehyd

g) Reduktion: → $II_1$; 1-Octadecyl-2-allyl-sn-glycerin

$II_1$:h) Umlagerung → $II_2$; 1-Octadecyl-2-propenyl-sn-glycerin

E) <u>Einführung von Alkenylresten in die sn-1-Position des Glycerins</u> (natürliche Konfiguration)

(I')

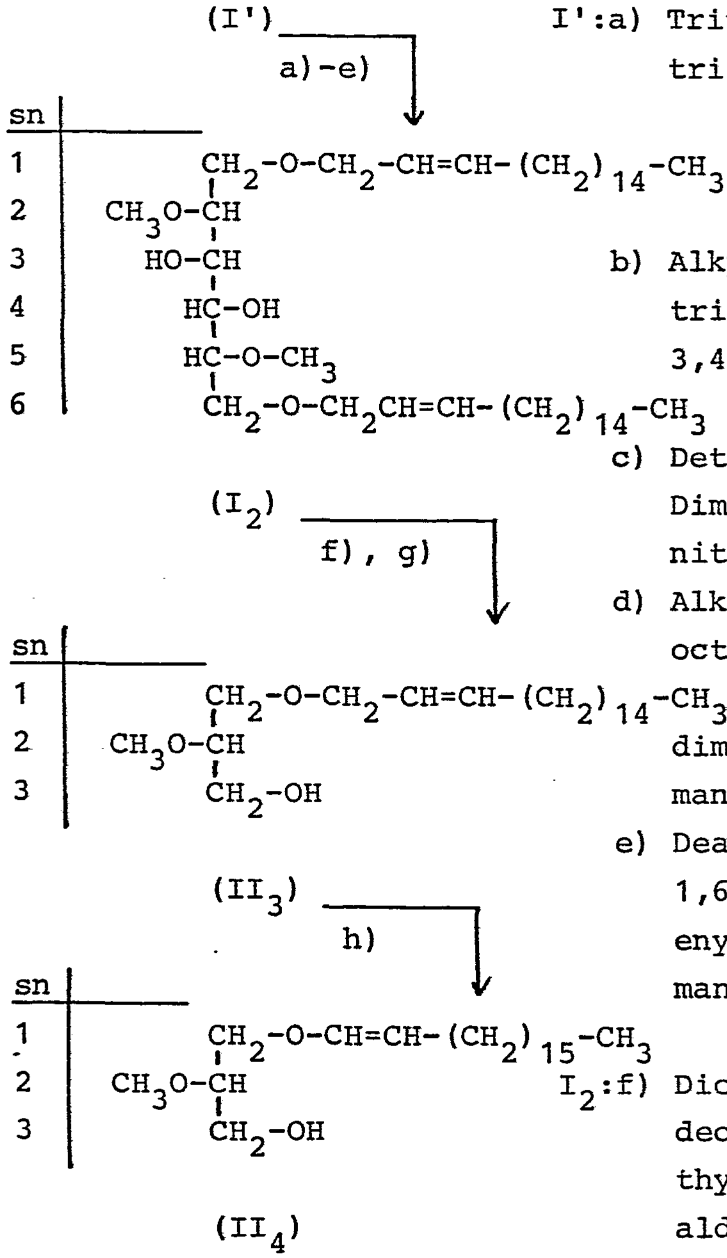


I':a) Tritylierung → 1,6-Di-trityl-3,4-Jp-D-mannit

b) Alkylierung → 1,6-Di-trityl-2,5-dimethyl-3,4-Jp-D-mannit

c) Detritylierung → 2,5-Dimethyl-3,4-Jp-D-mannit

d) Alkylierung → 1,6-Di-octadec-(2')-enyl-2,5-dimethyl-3,4-Jp-D-mannit

e) Deacetonierung: → $I_2$; 1,6-Dioctadec-(2')-enyl-2,5-dimethyl-D-mannit

$I_2$:f) Diolspaltung → 1-Octa-dec-(2')-enyl-2-me-thyl-sn-glycerin-3-aldehyd

g) Reduktion → $II_3$; 1-Oc-tadec-(2')-enyl-2-me-thyl-sn-glycerin

$II_3$:h) Umlagerung → $II_4$; 1-Octadec-(1')-enyl-2-methyl-sn-glycerin

F) Phospholipide aus Diätherglycerinen

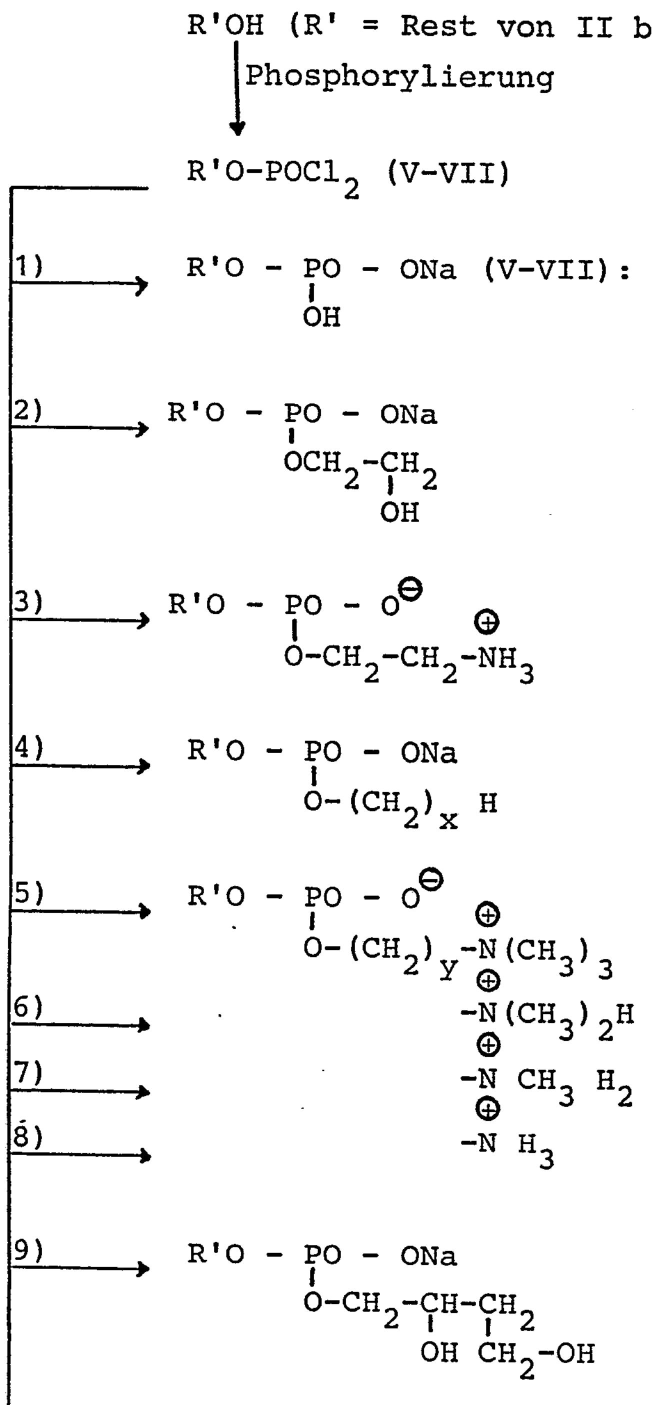


1) Hydrolyse → Phosphatidsäuren ($VIII_1$-$X_1$)

2) Glykol; Hydrolyse → Phosphatidylglykole ($VIII_2$-$X_2$)

3) Äthanolamin; saure Hydrolyse → Phosphatidyläthanolamine ($VIII_3$-$X_3$)

4) Alkanole (x = 1 - 18) Hydrolyse → Phosphatidsäurealkylester ($VIII_4$-$X_4$)

5) Bromalkanole (y = 2 - 12); Hydrolyse; Aminierung → Phosphatidylcholine ($VIII_5$-$X_5$)

6) N,N-Dimethyl-Phosphatidyläthanolamine ($VIII_6$-$X_6$)

7) N-Methyl-phosphatidyläthanolamin ($VIII_7$-$X_7$)

8) Phosphatidyläthanolamine ($VIII_8$-$X_8$)

9) 1,2-Isopropylidenglycerin; basische und saure Hydrolyse → Phosphatidylglycerin ($VIII_9$-$X_9$)

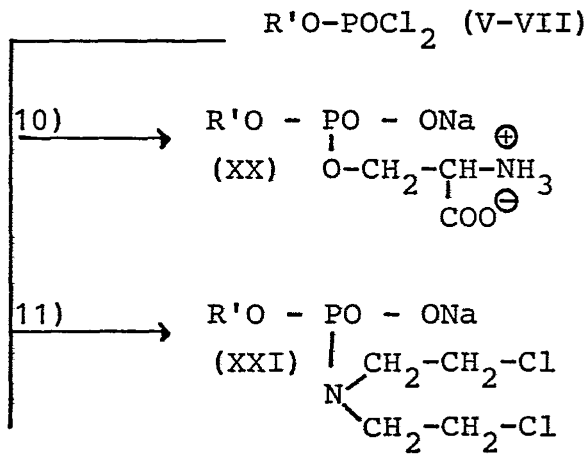


(XX)

(XXI)

10) Hydrolyse; N-Carbobenzoxy-serin-benzylester; kat. Hydrogenolyse → Phosphatidylserin ($V_7$-$VII_7$)

11) Amine; Hydrolyse: → Phosphatidsäureamid ($V_8$-$VII_8$)

B e i s p i e l e

Stereo- und positionsspezifische Synthese von Diätherglycerinen (gesättigte Kohlenwasserstoffreste, Reaktionsschemen A bis C)

Tritylierung A - C, a:

Ausgangsprodukt ist das nach L. F. Wiggins (J. Chem. Soc. 13, 1946) gut zugängliche 3,4-Isopropyliden-D-mannit (I'). Eine Lösung von I' (0,1 Mol) in 500 ml tert. Butanol wird mit Tritylchlorid (0,2 Mol) und Triäthylamin (0,3 Mol) versetzt. Man kocht unter Rückfluß bis zur Vollständigkeit der Reaktion (DC-Kontrolle). Die Hauptmenge an tert. Butanol wird im Vakuum abgezogen, der Rückstand in 500 ml Diisopropylidenäther aufgenommen und mit Wasser extrahiert. Die Diisopropylätherphase wird einrotiert und der ölige Rückstand, vorwiegend 1,6-Ditrityl-3,4-Isopropyliden-D-mannit (0,1 Mol), in 1 l Dioxan aufgenommen (Lösung A - C, a).

Alkylierung A - C, b:

Die Lösung A - C, a (0,1 Mol) wird mit Kalium-tert.-butylat versetzt (0,2 Mol) und unter Rückfluß gekocht. Unter Rühren wird Methyljodid oder Dimethylsulfat (0,3 Mol) eingetropft (A und B, b) oder mit Benzylchlorid versetzt (C, b). Man kocht bis zur Vollständigkeit der Reaktion (DC-Kontrolle) und versetzt mit 1 l Diisopropyläther. Nach Extraktion der Diisopropylätherphase mit Wasser wird diese einrotiert und der Rückstand an Kieselgel durch Chromatographie gereinigt. Man erhält in den Reaktionen A, B-b 1,6-Ditrityl-2,5-dimethyl-3,4-isopropyliden-D-mannit, und in C, b 1,6-Ditrityl-2,4-dibenzyl-3,4-isopropyliden-D-mannit, in Ausbeuten von 85 - 90 %.

Detritylierung A - C, c:

Die säurelabile Tritylgruppe wird in Gegenwart von Schwefelsäure und Aceton entfernt. Die jeweilige Ditritylverbindung (A - C, b; 0,1 Mol) wird dazu in 500 ml Aceton gelöst, mit 500 ml Methanol versetzt, das 5 ml konzentrierte Schwefelsäure enthält, und auf 50°C im Wasserbad erwärmt. Nach 60 Minuten sind die Tritylreste aus der 1,6-Position abgespalten. Man versetzt mit 1 l Diisopropylidenäther und extrahiert zweimal mit je 1 l Wasser, zum Schluß mit 1 l wäßrigem 1M $NaHCO_3$. Die Diisopropylätherphase wird einrotiert und an Kieselgel gereinigt. Man erhält A, B-c, 2,5-Dimethyl-3,4-Isopropyliden-D-mannit und C, c, 2,5-Dibenzyl-3,4-isopropyliden-D-mannit, in Ausbeuten von 90 - 95 %.

Alkylierung A - C, d:

Zur Einführung der Alkyl- oder Benzylreste in 1,6-Position werden die Zwischenprodukte A - C, c, jeweils 0,1 Mol, in 500 ml Toluol gelöst und mit Kalium-tert.-Butylat (0,2 Mol) versetzt. Man kocht unter Rückfluß und tropft unter Rühren jeweils 0,2 Mol der entsprechenden Verbindungen ein; zum Beispiel zur Darstellung von A, d Octadecylmesylat-bromid oder -jodid, zur Darstellung von B, d Benzylchlorid oder -bromid und zur Darstellung von C, d Methyljodid oder Dimethylsulfat. Nach Abschluß der Reaktion (DC-Kontrolle) wird die Toluolphase zweimal mit Wasser extrahiert und einrotiert. Der Rückstand aus Reaktion A, d ist hauptsächlich 1,6-Dioctadecyl-2,5-dimethyl-3,4-isopropyliden-D-mannit, aus B, d 1,6-Dibenzyl-2,5-dimethyl-3,4-isopropyliden-D-mannit, oder aus C, d 1,6-Dimethyl-2,5-dibenzyl-3,4-isopropyliden-D-mannit; Ausbeuten ca. 90 %.

Deacetonierung A - C, e:

Zur Entfernung der Isopropylidengruppe in 3,4-Position der Mannitderivate verwendet man wäßrige Schwefelsäure

in 2-Propanol/Methanol 1:1. Das jeweilige Zwischenprodukt (0,1 Mol) aus A - C, d wird in 1 l des Lösungsmittelgemisches mit 50 ml 2N-$H_2SO_4$ versetzt und unter Rückfluß bis zur vollständigen Abspaltung der Isopropylidengruppe gekocht (DC-Kontrolle). Man versetzt mit 1 l Diisopropyläther, extrahiert zweimal mit jeweils 1 l Wasser und rotiert die Diisopropylidenätherphase ein. Die Zwischenprodukte $A_I$, $B_I$ und $C_I$ werden chromatographisch an Kieselgel gereinigt und durch Mikroanalyse charakterisiert; Ausbeuten 90 - 95 %.

Auf diese Weise wurden die nachstehenden Verbindungen der Formel I dargestellt:

1,6-Dioctadecyl-2,5-dimethyl-D-mannit
($C_{44}H_{90}O_6$; 715,20)

ber: C 73,89 H 12,69
gef: C 74,01 H 12,84

1,6-Ditetradecyl-2,5-dimethyl-D-mannit
($C_{36}H_{74}O_6$; 602,99)

ber: C 71,71 H 12,37
gef: C 71,76 H 12,42

1,6-Didocosanyl-2,5-dimethyl-D-mannit
($C_{52}H_{106}O_6$; 827,42)

ber: C 75,48 H 12,91
gef: C 75,69 H 12,94

1,6-Dioctadecyl-2,5-dipentyl-D-mannit
($C_{52}H_{106}O_6$; 827,42)

ber: C 75,48 H 12,91
gef: C 75,84 H 13,01

1,6-Dioctadecyl-2,5-didodecyl-D-mannit
($C_{66}H_{134}O_6$; 1023,798)

ber: C 77,43 H 13,19
gef: C 77,41 H 13,27

1,6-Dibenzyl-2,5-dimethyl-D-mannit
($C_{22}H_{30}O_6$; 390,48)

ber: C 67,67 H 7,74
gef: C 67,66 H 7,76

1,6-Dimethyl-2,5-dibenzyl-D-mannit
($C_{22}H_{30}O_6$; 390,48)

ber: C 67,67 H 7,74
gef: C 67,82 H 7,91

Diolspaltung A - C, f:

Die Zwischenprodukte der Formel I (jeweils 0,1 Mol) werden in 500 ml Benzol gelöst und portionsweise mit Bleitetraacetat (ca. 0,1 Mol) versetzt, bis das Ausgangsprodukt nicht mehr nachzuweisen ist (DC-Kontrolle). Das Reaktionsgemisch wird zweimal mit jeweils 500 ml Wasser gewaschen, die Benzolphase einrotiert und der Rückstand in 500 ml Methanol aufgenommen.

Reduktion A - C, g:

Die Lösung der Aldehyde in Methanol wird portionsweise mit $NaBH_4$ versetzt (ca. 0,1 Mol), bis der Aldehydnachweis negativ ausfällt. Man versetzt mit 500 ml Diisopropyläther und schüttelt zweimal mit Wasser aus. Die Diäthylätherphase wird einrotiert und der Rückstand an Kieselgel chromatographiert. Man erhält die Verbindungen der Formel II in Ausbeuten von 90 %.

Auf diese Weise wurden die nachstehenden Verbindungen der Formel II dargestellt:

1-Octadecyl-2-methyl-sn-glycerin
($C_{22}H_{46}O_3$; 358,61)

ber: C 73,69 H 12,93
Gef: C 73,71 H 12,96

1-Tetradecyl-2-methyl-sn-glycerin
($C_{18}H_{38}O_3$; 302,502)

ber: C 71,47 H 12,66
gef: C 71,55 H 12,70

1-Docosanyl-2-methyl-sn-glycerin
($C_{26}H_{54}O_3$; 414,72)

ber: C 75,30 H 13,13
gef: C 75,35 H 13,13

1-Octadecyl-2-pentyl-sn-glycerin
($C_{26}H_{54}O_3$; 414,72)

ber: C 75,30 H 13,13
gef: C 75,37 H 13,17

1-Octadecyl-2-dodecyl-sn-glycerin
($C_{33}H_{68}O_3$; 512,91)

ber: C 77,28 H 13,36
gef: C 77,35 H 13,31

1-Benzyl-2-methyl-sn-glycerin
($C_{11}H_{16}O_3$; 196,25)

ber: C 67,32 H 8,22
gef: C 67,46 H 8,27

1-Methyl-2-benzyl-sn-glycerin
($C_{11}H_{16}O_3$; 196,25)

ber: C 67,32 H 8,22
gef: C 67,39 H 8,23

Alkylierung B oder C, h:

Die Verbindung A II steht direkt zur Phosphorylierung zur Verfügung. In die Verbindungen B II und C II werden vor der Phosphorylierung noch Octadecylreste eingeführt und die gewünschte Position im Glycerinmolekül freigelegt. Dazu werden die Zwischenprodukte B II und C II (jeweils 0,1 Mol) in 500 ml Toluol gelöst und mit Kalium-tert.-butylat (0,1 Mol) versetzt. Man kocht unter Rückfluß und tropft unter Rühren eine Lösung von Octadecylmesylat (jeweils 0,1 Mol) in 100 ml Toluol ein und erhält aus Reaktion B, h 1-Benzyl-2-methyl-3-octadecyl-sn-glycerin und aus Reaktion C, h 1-Methyl-2-benzyl-3-octadecyl-sn-glycerin. Nach Beendigung der Reaktion (DC-Kontrolle) wird die Toluolphase zweimal mit Wasser extrahiert, einrotiert und der Rückstand durch Chromatographie an Kieselgel gereinigt. Die Ausbeuten liegen bei 95 %.

Katalytische Debenzylierung B oder C, i:

Die Produkte aus der Reaktion B oder C, h (0,1 Mol) werden in 200 ml Diisopropyläther gelöst, mit 4 g Pd/C (10 %) und 0,4 g Palladiumschwarz versetzt und solange unter Rühren in einer $H_2$-Atmosphäre belassen,

bis die $H_2$-Aufnahme abgeschlossen ist. Die Verbindungen B III und C IV entstehen quantitativ und können direkt nach Entfernen des Diisopropyläthers zur Phosphorylierung verwendet werden.

Auf diese Weise wurden die nachstehenden Verbindungen dargestellt:

Verbindungen der Formel III:

3-Octadecyl-2-methyl-sn-glycerin
($C_{22}H_{46}O_3$; 358,61)

ber: C 73,69 H 12,93
gef: C 73,82 H 12,95

3-Octadecyl-2-pentyl-sn-glycerin
($C_{26}H_{54}O_3$; 414,72)

ber: C 75,30 H 13,13
gef: C 75,45 H 13,19

3-Octadecyl-2-dodecyl-sn-glycerin
($C_{33}H_{68}O_3$; 512,91)

ber: C 77,28 H 13,36
gef: C 77,35 H 13,41

Verbindungen der Formel IV:

3-Octadecyl-1-methyl-sn-glycerin
($C_{22}H_{46}O_3$; 358,61)

ber: C 73,69 H 12,93
gef: C 73,79 H 12,98

3-Octadecyl-1-pentyl-sn-glycerin
($C_{26}H_{54}O_3$; 414,72)

ber: C 75,30 H 13,13
gef: C 75,25 H 13,11

3-Octadecyl-1-dodecyl-sn-glycerin
($C_{33}H_{68}O_3$; 512,91)

ber: C 77,28 H 13,36
gef: C 77,27 H 13,45

Einführung von Alkenylresten in die sn-2- oder sn-1-Position des Glycerins (Reaktionsschemen D und E)

Die einzelnen Reaktionsschritte sind in den Reaktionsschemen D und E dargestellt und systematisch unterteilt in Einführung von Alkenylresten in die sn-2-Position des Glycerins und Einführung von Alkenylresten in die sn-1-Position des Glycerins. Für die Einführung von Alkinylresten müssen die gleichen Vorsichtsmaßnahmen wie für die Alkenylreste beachtet werden. Die wichtigste Einschränkung besteht darin, daß Benzylreste als Schutzgruppen nicht verwendet werden können. Für die Positionierung der Reste in 1- und 2-Position ist aber die Tritylschutzgruppe ausreichend, und das führt zu den interessanten Vertretern mit natürlicher Konfiguration, d. h. freier Hydroxylgruppe in sn-3-Position. Eine Konfigurationsumkehr nach Reaktionsschema B h, i ist ebenfalls leicht möglich durch Ersatz der Benzylgruppe durch Trityl. In Reaktionsschema C h, i kann jedoch Benzyl nicht durch Trityl ersetzt werden. Man kann hier auf die Allylschutzgruppe ausweichen.

Die einzelnen Reaktionsschritte werden auch hier wieder anhand spezieller Beispiele erläutert. Es wird die Synthese von 1-Octadecyl-2-allyl-sn-glycerin und von 1-Octadecyl-2-propenyl-sn-glycerin (Reaktionsschema D) sowie von 1-Octadec-(2')-enyl-2-methyl-sn-glycerin und von 1-Octadec-(1')-enyl-2-methyl-sn-glycerin (Reaktionsschema E) beschrieben.

Tritylierung D oder E, a:

Ausgangsprodukt ist das nach L. F. Wiggins (J. Chem. Soc. 13, 1946) gut zugängliche 3,4-Isopropyliden-D-mannit (I'). Eine Lösung von I' (0,1 Mol) in 500 ml tert. Butanol wird mit Tritylchlorid (0,2 Mol) und Triäthylamin (0,3 Mol) versetzt. Man kocht unter Rückfluß bis zur Vollständigkeit der Reaktion (DC-Kontrolle). Die Hauptmenge an tert. Butanol wird im Vakuum abgezogen, der Rückstand in 500 ml Diisopropylidenäther aufgenommen und mit Wasser extrahiert. Die Diisopropylätherphase wird einrotiert und der ölige Rückstand, vorwiegend 1,6-Ditrityl-3,4-isopropyliden-D-mannit (0,1 Mol), in 1 l Dioxan aufgenommen (Lösung D oder E, a).

Alkylierung D oder E, b:

Die Lösung D oder E, a (0,1 Mol) wird mit Kalium-tert.-butylat versetzt (0,2 Mol) und unter Rückfluß gekocht. Unter Rühren wird Allylchlorid (0,3 Mol) eingetropft (D, b) oder mit Methyljodid versetzt (E, b). Man kocht bis zur Vollständigkeit der Reaktion (DC-Kontrolle) und versetzt mit 1 l Diisopropyläther. Nach Extraktion der Diisopropylätherphase mit Wasser wird diese einrotiert und der Rückstand an Kieselgel durch Chromatographie gereinigt. Man erhält nach D, b 1,6-Ditrityl-2,5-diallyl-3,4-isopropyliden-D-mannit, und nach E, b 1,6-Ditrityl-2,5-dimethyl-3,4-isopropyliden-D-mannit, in Ausbeuten von 85 - 90 %.

Detritylierung C oder E, c:

Die säurelabile Tritylgruppe wird in Gegenwart von Schwefelsäure und Aceton entfernt. Die jeweilige Ditritylverbindung (D, E, b; 0,1 Mol) wird dazu in 500 ml Aceton gelöst, mit 500 ml Methanol versetzt, das 5 ml konzentrierte Schwefelsäure enthält, und auf 50 °C im Wasserbad erwärmt. Nach 60 Minuten sind die Tritylreste aus der 1,6-Position abgespalten. Man versetzt mit 1 l Diisopropylidenäther und extrahiert zweimal mit je 1 l Wasser, zum Schluß mit 1 l wäßrigem 1 M $NaHCO_3$. Die Diisopropylätherphase wird einrotiert und an Kieselgel gereinigt. Man erhält nach D, c 2,5-Diallyl-3,4-isopropyliden-D-mannit und nach E, c 2,5-Dimethyl-3,4-isopropyliden-D-mannit, in Ausbeuten von 90 - 95 %.

Alkylierung D oder E, d:

Zur Einführung der Substituenten in 1,6-Position werden die Verbindungen D, E - c, jeweils 0,1 Mol, in 500 ml Toluol gelöst und mit Kalium-tert.-butylat (0,2 Mol) versetzt. Man kocht unter Rückfluß und tropft unter Rühren jeweils 0,2 Mol der entsprechenden Verbindungen ein; zum Beispiel zur Darstellung von D, d Octadecylmesylat-bromid oder -jodid, zur Darstellung von E, d Octadec-(2')-enylmesylat oder -bromid. Nach Abschluß der Reaktion (DC-Kontrolle) wird die Toluolphase zweimal mit Wasser extrahiert und einrotiert. Der Rückstand aus Reaktion D, d ist hauptsächlich 1,6-Dioctadecyl-2,5-diallyl-3,4-isopropyliden-D-mannit, aus E, d 1,6-Dioctadec-(2')-enyl-2,5-dimethyl-3,4-isopropyliden-D-mannit in Ausbeuten von ca. 90 %.

Deacetonierung D oder E, e:

Zur Entfernung der Isopropylidengruppe in 3,4-Position der Mannitderivate verwendet man wäßrige Schwefelsäure

in 2-Propanol/Methanol 1:1. Das jeweilige Zwischenprodukt (0,1 Mol) aus D, E-d wird in 1 l des Lösungsmittelgemisches mit 50 ml 2N-$H_2SO_4$ versetzt und unter Rückfluß bis zur vollständigen Abspaltung der Isopropylidengruppe gekocht (DC-Kontrolle). Man versetzt mit 1 l Diisopropyläther, extrahiert zweimal mit jeweils 1 l Wasser und rotiert die Diisopropylidenätherphase ein. Die Zwischenprodukte werden chromatographisch an Kieselgel gereinigt und durch Mikroanalyse charakterisiert. Man erhält D, I, 1,6-Dioctadecyl-2,5-diallyl-D-mannit, und E, I, 1,6-Dioctadec-(2')-enyl-2,5-dimethyl-D-mannit, in Ausbeuten von ca. 90 %.

Auf diese Weise wurden die nachstehenden Verbindungen der Formel I dargestellt:

1,6-Dioctadecyl-2,5-diallyl-D-mannit
($C_{48}H_{96}O_6$; 769,30)

ber: C 74,94 H 12,58
gef: C 74,51 H 12,31

1,6-Dioctadecyl-2,5-dipropargyl-D-mannit
($C_{48}H_{94}O_6$; 719,24)

ber: C 75,14 H 12,35
gef: C 74,91 H 11,98

1,6-Dioctadec-(2')-enyl-2,5-dimethyl-D-mannit
($C_{44}H_{88}O_6$; 713,19)

ber: C 74,10 H 12,44
gef: C 73,89 H 12,28

1,6-Dioctadec-(2')-enyl-2,5-dipropyl-D-mannit
($C_{48}H_{96}O_6$; 769,30)

ber: C 74,94 H 12,58
gef: C 74,61 H 12,31

1,6-Diallyl-2,5-dioctadecyl-D-mannit
($C_{48}H_{96}O_6$; 769,30)

ber: C 74,94 H 12,58
gef: C 74,39 H 12,41

1,6-Dioctadec-(9')-enyl-2,5-dimethyl-D-mannit
($C_{44}H_{88}O_6$; 713,19)

ber: C 74,10 H 12,44
gef: C 73,71 H 12,23

1,6-Dioctadecyl-2,5-dioctadec-(9')-enyl-D-mannit
($C_{78}H_{154}O_6$; 1188,09)

ber: C 78,85 H 13,07
gef: C 78,49 H 12,86

Diolspaltung D, E - f:
Die Zwischenprodukte D, I und E, I (jeweils 0,1 Mol) werden in 500 ml Benzol gelöst und portionsweise mit Bleitetraacetat (ca. 0,1 Mol) versetzt, bis das Ausgangsprodukt nicht mehr nachzuweisen ist (DC-Kontrolle). Das Reaktionsgemisch wird zweimal mit jeweils 500 ml Wasser gewaschen, die Benzolphase einrotiert und der Rückstand in 500 ml Methanol aufgenommen.

Reduktion D, E - g:
Die Lösung der Aldehyde in Methanol wird portionsweise

mit $NaBH_4$ versetzt (ca. 0,1 Mol), bis der Aldehydnachweis negativ ausfällt. Man versetzt mit 500 ml Diisopropyläther und schüttelt zweimal mit Wasser aus. Die Diäthylätherphase wird einrotiert und der Rückstand an Kieselgel chromatographiert. Man erhält D, II, 1-Octadecyl-2-allyl-sn-glycerin und E, II, 1-Octadecyl-(2')-enyl-2-methyl-sn-glycerin in Ausbeuten von etwa 95 %.

Umlagerung D, E - h:
Die Reaktionsprodukte D, II und E, II (0,1 Mol) werden in 20 ml Dimethylformamid gelöst und mit K-tert.-butylat versetzt (0,15 Mol). Man erhitzt so lange auf 100 bis 110°C (ca. 60 Min.), bis die Verschiebung der Doppelbindung vollständig ist. Man kühlt auf 20°C, versetzt mit 200 ml Diisopropyläther und extrahiert mit 200 ml Wasser. Die Diäthylätherphase wird einrotiert und an Kieselgel chromatographiert. Man erhält D, II 1-Octadecyl-2-propenyl-sn-glycerin, in Ausbeuten von ca. 90 %.

Auf diese Weise wurden die nachstehenden Verbindungen der Formel II dargestellt:

1-Octadecyl-2-allyl-sn-glycerin
($C_{24}H_{48}O_3$; 384,65)

ber: C 74,94 H 12,58
gef: C 74,69 H 12,44

1-Octadec-(9')-enyl-2-propyl-sn-glycerin
($C_{24}H_{48}O_3$; 384,65)

ber: C 74,94 H 12,58
gef: C 74,63 H 12,51

0098600

1-Octadec-(2')-enyl-2-methyl-sn-glycerin
($C_{22}H_{44}O_3$; 356,59)

ber: C 74,10 H 12,44
gef: C 73,89 H 12,37

1-Octadec-(1')-enyl-2-methyl-sn-glycerin
($C_{22}H_{44}O_3$; 356,59)

ber: C 74,10 H 12,44
gef: C 73,94 H 12,21

1-Octadec-(1')-enyl-2-dodecyl-sn-glycerin
($C_{33}H_{66}O_3$; 510,89)

ber: C 77,58 H 13,02
gef: C 77,06 H 12,82

Die in den Diäthylglycerinen vorliegenden freien Hydroxylgruppen (sn-3-Hydroxyl für A, D, E-II; sn-1-Hydroxyl für B III und sn-2-Hydroxyl für C, IV) werden nach einem allgemeinen Verfahren phosphoryliert und in ein Phosphorsäuredichlorid umgewandelt, das das zentrale Zwischenprodukt für die Einführung der Reste $R_3$ darstellt (siehe dazu Reaktionsschema F: Phospholipide aus Diätherglycerinen). Da die Strukturmodifikation auf dieser Stufe der Synthese nur noch in $R_3$, allerdings in unterschiedlicher Verknüpfung mit dem Phosphor, erfolgt, werden zur Vereinheitlichung die unterschiedlichen Grundkörper A II, B III und C IV unter dem Symbol R' zusammengefaßt. Das Reaktionsschema F beschreibt beispielhaft einige typische Reaktionen, die zur Einführung des Restes $R_3$ dienen.

Phosphatidsäurechloride V - VII:

Die Zwischenverbindungen II - IV, beispielsweise aus A - C (jeweils 0,1 Mol) werden in 500 ml THF aufgenommen und mit 0,2 Mol Triäthylamin versetzt. Man gibt die Lösung bei 20° tropfenweise und unter Rühren zu Phosphoroxychlorid (0,15 Mol) in 100 ml THF. Nach Beendigung der Reaktion (DC-Kontrolle) wird das ausgefallene Triäthylaminhydrochlorid abfiltriert, mit 100 ml Toluol versetzt und am Rotationsverdampfer eingeengt (Entfernung von überschüssigem $POCl_3$). Man erhält einen öligen Rückstand, der hauptsächlich aus 1-Octadecyl-2-methyl-sn-glycero-3-phosphodichlorid (V), 3-Octadecyl-2-methyl-sn-glycero-1-phosphodichlorid (VI) oder 1-Methyl-3-octadecyl-sn-glycero-3-phosphodichlorid (VII) besteht und in 400 ml THF aufgenommen wird. Die Ausbeute liegt bei 95 %.

Phosphatidsäuren $VIII_1$ - $X_1$:

Die THF-Lösungen von V - VII werden bei 10°C jeweils mit 80 ml Wasser versetzt und solange gerührt, bis die Hydrolyse vollständig ist. Man versetzt mit 300 ml Wasser und extrahiert mit 300 ml Chloroform. Die Chloroformphase wird mit 300 ml Methanol versetzt und mit 300 ml Natriumhydrocarbonat ausgeschüttelt. Nach Entfernen des Chloroforms wird der Rückstand an Kieselgel chromatographiert. Man erhält Endprodukte in Ausbeuten von 90 %, bezogen auf die Diätherglycerine, und zwar 1-Octadecyl-2-methyl-sn-glycero-3-phosphat ($VIII_1$), 3-Octadecyl-2-methyl-sn-glycero-1-phosphat ($IX_1$) und 1-Methyl-3-octadecyl-sn-glycero-2-phosphat ($X_1$).

Auf diese Weise wurden die nachstehenden Verbindungen der Formel $VIII_1$ - $X_1$ dargestellt:

1-Octadecyl-2-methyl-sn-glycero-3-phosphat, Natriumsalz
($C_{22}H_{46}O_6PNa$; 460,58)

ber: C 57,37 H 10,07 P 6,73
gef: C 57,55 H 10,13 P 6,50

3-Octadecyl-2-methyl-sn-glycero-1-phosphat, Natriumsalz
($C_{22}H_{46}O_6PNa$; 460,55)

ber: C 57,37 H 10,07 P 6,73
gef: C 58,01 H 10,27 P 6,67

1-Methyl-3-octadecyl-sn-glycerin-2-phosphat, Natriumsalz
($C_{22}H_{46}O_6PNa$; 460,58)

ber: C 57,37 H 10,07 P 6,73
gef: C 57,46 H 10,10 P 6,51

1-Pentyl-3-octadecyl-sn-glycerin-2-phosphat, Natriumsalz
($C_{26}H_{54}O_6PNa$; 516,68)

ber: C 60,44 H 10,54 P 4,45
gef: C 60,50 H 10,77 P 4,23

1-Dodecyl-3-octadecyl-sn-glycerin-2-phosphat, Natriumsalz
($C_{33}H_{68}O_6PNa$; 614,87)

ber: C 64,46 H 11,15 P 3,74
gef: C 64,67 H 11,19 P 3,61

Phosphatidylglykole $VIII_2 - X_2$:
Die THF-Lösungen von V - VII (0,1 Mol in 400 ml THF) werden tropfenweise bei 20°C mit einer Lösung von Triäthylamin (0,4 Mol) in 100 ml THF und dann mit Äthylenglykol (0,2 Mol) in 300 ml THF versetzt. Man erwärmt

auf 40°C. Nach Beendigung der Reaktion (DC-Kontrolle) wird von Triäthylaminhydrochlorid abfiltriert und mit 100 ml Wasser zur Hydrolyse des Zwischenproduktes versetzt. Man gibt weitere 300 ml Wasser zu und extrahiert mit Chloroform. Der Rückstand aus der Chloroformphase wird an Kieselgel chromatographiert. Man erhält $VIII_2$, 1-Octadecyl-2-methyl-sn-glycero-3-phosphoglykol und die entsprechenden Verbindungen $IX_2$ und $X_2$ in Ausbeuten von ca. 90 %.

<u>Verbindungen der Formel $VIII_2$ - $X_2$:</u>

1-Octadecyl-2-Methyl-sn-glycero-3-phosphoglykol, Natriumsalz
($C_{24}H_{50}NaO_7P$; 504,63)

ber: C 57,12 H 9,99 P 6,14
gef: C 56,81 H 9,87 P 6,07

3-Octadecyl-2-methyl-sn-glycerin-2-phosphoglykol, Natriumsalz
($C_{24}H_{50}NaO_7P$; 504,63)

ber: C 57,12 H 9,99 P 6,14
gef: C 57,01 H 9,76 P 5,93

1-Octadec-(2')-enyl-2-methyl-sn-glycerin-2-phosphoglykol, Natriumsalz
($C_{24}H_{48}NaO_7P$; 502,61)

ber: C 57,35 H 9,63 P 6,16
gef: C 57,21 H 9,51 P 5,88

<u>Phosphatidyläthanolamine $VIII_3$ - $X_3$:</u>

Die THF-Lösungen von V - VII (0,1 Mol in 400 ml THF) werden tropfenweise bei 20°C mit einer Lösung von Triäthylamin (0,4 Mol) in 100 ml THF und dann mit Äthanolamin (0,2 Mol) in 300 ml THF versetzt. Man erwärmt auf 40°C. Nach Beendigung der Reaktion (DC-Kontrolle) wird vom Triäthylaminhydrochlorid abfiltriert, mit 100 ml Toluol versetzt und einrotiert. Der Rückstand, das Oxazaphospholan von II, III oder IV, wird in 300 ml 2-Propanol aufgenommen und mit 150 ml 20 % Ameisensäure auf 60°C erwärmt. Nach 60 Min. ist die Ringöffnung vollständig. Die Phosphatidyläthanolamine der entsprechenden Struktur ($VIII_3$ - $X_3$) fallen aus und werden zur vollständigen Reinigung noch an Kieselgel chromatographiert. Die Ausbeuten betragen etwa 90 % in bezug auf die entsprechenden Alkohole.

<u>Verbindungen der Formel $VIII_3$ - $X_3$:</u>

1-Octadecyl-2-methyl-sn-glycero-3-phosphoäthanolamin ($C_{24}H_{52}NO_6P$; 481,66)

ber: C 59,85 H 10,88 P 6,43
gef: C 60,01 H 10,91 P 6,31

3-Octadecyl-2-methyl-sn-glycero-2-phosphoäthanolamin ($C_{24}H_{52}NO_6P$; 481,66)

ber: C 59,85 H 10,88 P 6,43
gef: C 59,74 Z 10,74 P 6,37

<u>Phosphatidsäurealkylester $VIII_4$ - $X_4$:</u>

Die THF-Lösungen von V - VII (0,1 Mol in 400 ml THF) werden tropfenweise bei 20°C mit einer Lösung von Triäthylamin (0,4 Mol) in 100 ml THF und dann mit Alkohol (0,2 Mol) in 300 ml THF versetzt. Man erwärmt auf 40°C. Nach Beendigung der Reaktion wird vom Triäthylaminhydrochlorid abfiltriert und zur Hydrolyse mit 100 ml $H_2O$ versetzt. Man rührt bis zur Beendigung der

Reaktion (DC-Kontrolle), versetzt mit 400 ml Wasser und extrahiert mit Chloroform. Der Rückstand aus der Chloroformphase wird noch an Kieselgel chromatographiert. Man erhält die Produkte $VIII_4$ - $X_4$ in Ausbeuten von etwa 90 %.

<u>Verbindungen der Formeln $VIII_4$ - $X_4$:</u>

1-Octadecyl-2-methyl-sn-glycero-3-phosphorsäuremethylester, Natriumsalz
($C_{23}H_{48}NaO_6P$; 474,60)

ber: C 58,21 H 10,20 P 6,53
gef: C 58,16 H 10,04 P 6,41

1-Octadecyl-2-methyl-sn-glycero-3-phosphorsäureoctylester, Natriumsalz
($C_{30}H_{62}NaO_6P$; 572,60)

ber: C 62,91 H 10,91 P 5,41
gef: C 62,56 H 10,82 P 5,42

<u>Phosphatidylcholine $VIII_5$ - $X_5$:</u>

Die für die Darstellung von Phosphatidylcholinen notwendigen Bromalkyl-Phosphatidsäuren werden aus Bromalkanolen analog $VIII_4$ - $V_4$ erhalten. Zur Aminierung werden die Bromalkylester der entsprechenden Phosphatidsäuren (0,1 Mol) in 100 ml Chloroform gelöst und mit 100 ml 2-Propanol versetzt, das 30 g Trimethylamin enthält. Nach 8 Stunden bei 50°C wird gegen Wasser ausgeschüttelt, die Chloroformphase einrotiert und der Rückstand an Kieselgel chromatographiert. Die erwarteten Produkte entstehen in etwa 90 % Ausbeute.

<u>Verbindungen der Formel $VIII_5$ - $X_5$:</u>

1-Octadecyl-2-methyl-sn-glycerin-3-phosphocholin
($C_{27}H_{58}NO_6P$; 523,74)

ber: C 61,92 H 11,16 P 5,91
gef: C 61,95 H 11,18 P 5,76

1-Octadecyl-2-methyl-sn-glycero-3-phospho-N,N,N-tri-methyl-hexanolamin
($C_{31}H_{66}NO_6P$; 579,85)

ber: C 64,21 H 11,47 P 5,34
gef: C 64,33 H 11,48 P 5,29

1-Octadecyl-2-pentyl-sn-glycerin-3-phosphocholin
($C_{31}H_{66}NO_6P$; 579,85)

ber: C 64,21 H 11,47 P 5,34
gef: C 64,49 H 11,55 P 5,17

1-Octadecyl-2-dodecyl-sn-glycero-3-phosphocholin
($C_{38}H_{80}NO_6P$; 678,04)

ber: C 67,31 H 11,89 P 4,57
gef: C 67,46 H 11,94 P 4,41

<u>Phosphatidyl-N,N-dimethyläthanolamine $VIII_6$ - $X_6$:</u>
Man verfährt wie unter $VIII_5$ - $X_5$ beschrieben, verwendet jedoch 30 g Dimethylamin in 100 ml Propanol im Aminierungsschritt.

Verbindungen der Formel $VIII_6$ - $X_6$:
1-Octadecyl-2-methyl-sn-glycerin-3-phospho-N,N-dimethyl-äthanolamin
($C_{26}H_{56}NO_6P$; 509,72)

ber: C 61,27 H 11,07 P 6,08
gef: C 61,35 H 11,14 P 5,96

Phosphatidyl-N-methyl-äthanolamine $VIII_7 - X_7$:

Man verfährt wie unter $VIII_5 - X_5$ beschrieben, verwendet aber 30 g Methylamin in 100 ml 2-Propanol im Aminierungsschritt.

Verbindungen der Formel $VIII_7 - X_7$:

1-Octadecyl-2-methyl-sn-glycero-3-phospho-N-methyläthanolamin

($C_{25}H_{54}NO_6P$; 495,69)

| | | | |
|---|---|---|---|
| ber: | C 60,58 | H 10,98 | P 6,25 |
| gef: | C 60,72 | H 11,05 | P 6,11 |

Phosphatidyläthanolamine $VIII_8 - X_8$ (= $VIII_3 - X_3$):

Man verfährt wie unter $VIII_5 - X_5$ beschrieben, verwendet aber 20 g $NH_3$ in 100 ml 2-Propanol im Aminierungsschritt.

Phosphatidylglycerine $VIII_9 - X_9$:

Die THF-Lösungen V - VII (0,1 Mol in 400 ml THF) werden bei 20°C tropfenweise mit einer Lösung von Triäthylamin (0,4 Mol) in 100 ml THF versetzt. Im Anschluß daran tropft man 1,2-Isopropyliden-glycerin (0,2 Mol) in 300 ml THF ein. Man erwärmt auf 40°C. Nach Beendigung der Reaktion (DC-Kontrolle) wird vom ausgefallenen Triäthylamin abfiltriert und das Filtrat bei 10°C mit 100 ml Wasser versetzt und solange gerührt, bis die Hydrolyse vollständig ist. Nach Zusatz von weiteren 100 ml Wasser wird der pH-Wert der wäßrigen Phase mit Ameisensäure auf pH 4 - 5 abgesenkt und solange gerührt, bis die Abspaltung der Isopropylidengruppe vollständig ist (DC-Kontrolle). Man extrahiert mit 300 ml Chloroform und behandelt die Chloroformphase mit 300 ml 1M-Natriumhydrogencarbonat. Die Chloroformphase wird einrotiert und der Rückstand an Kieselgel chromatographiert. Man erhält die gewünschten Produkte, $VIII_9$ - $X_9$, in etwa 90 % Ausbeute.

Verbindungen der Formel $VIII_9$ - $X_9$:

3-Octadecyl-2-methyl-sn-glycero-3-phosphoglycerin, Natriumsalz
($C_{25}H_{52}NaO_8P$; 534,66)

| | | | |
|---|---|---|---|
| ber: | C 56,16 | H 9,80 | P 5,79 |
| gef: | C 56,09 | H 9,51 | P 5,99 |

3-Octadecyl-2-pentyl-sn-glycero-3-phosphoglycerin, Natriumsalz
($C_{29}H_{60}NaO_8P$; 590,77)

| | | | |
|---|---|---|---|
| ber: | C 58,96 | H 10,24 | P 5,24 |
| gef: | C 58,51 | H 10,03 | P 5,01 |

3-Octadecyl-2-dodecyl-sn-glycero-3-phosphoglycerin, Natriumsalz
($C_{36}H_{74}NaO_8P$; 688,95)

| | | | |
|---|---|---|---|
| ber: | C 62,76 | H 10,83 | P 4,72 |
| gef: | C 63,04 | H 10,94 | P 4,53 |

<u>Phosphatidylserine $VIII_{10}$ - $X_{10}$:</u>

Die THF-Lösungen von V - VII werden wie in $VIII_1$ - $X_1$ beschrieben in Phosphatidsäuren umgewandelt (0,1 Mol). Das Natriumsalz der gereinigten Phosphatidsäuren (0,1 Mol) wird in 200 ml $CHCl_3$ gelöst, mit 200 ml Methanol versetzt und mit 200 ml =,1 N HCl ausgewaschen. Die Chloroformphase wird einrotiert und im Vakuum getrocknet. Die freie Phosphatidsäure (0,1 Mol) wird mittels handelsüblichem N-Carbobenzoxy-L-serin-benzylester (0,2 Mol) versetzt und 500 ml Pyridin zugegeben.

Dazu gibt man eine Lösung von käuflichem Triisopropylbenzolsulfonsäurechlorid (0,3 Mol) in 500 ml Pyridin und erwärmt auf 70°C, bis die Lösung klar wird. Nach 3 Stunden Rühren bei 20°C versetzt man mit 600 ml Chloroform und rührt für weitere 60 Minuten. Das Reaktionsgemisch wird dann mit 500 ml Wasser ausgewaschen und die Chloroformphase einrotiert. Man chromatographiert an Kieselgel; die geschützten Phosphatidylserine erhält man in etwa 50 % Ausbeute.

Die geschützten Zwischenprodukte (0,05 Mol) werden in 1 l Eisessig gelöst und mit 4 g Pd/C (10 %) und 0,4 g Palladiumschwarz versetzt. Man rührt solange in $H_2$-Atmosphäre, bis die Wasserstoffaufnahme aufhört. Die geschützten Phosphatidylserine gehen quantitativ in die freien Phosphatidylserine $VIII_{10}$ - $X_{10}$ über. Die Essigsäurelösung wird erwärmt, filtriert und mit 1 l Aceton versetzt. Man erhält eine chromatographisch einheitliche Substanz als feines, amorphes Pulver.

Verbindungen der Formeln $VIII_{10}$ - $X_{10}$:

1-Octadecyl-2-methyl-sn-glycero-3-phosphoserin, Natriumsalz

($C_{25}H_{51}NNaO_8P$; 547,66)

| | | | | |
|---|---|---|---|---|
| ber: | C 54,83 | H 9,39 | N 2,56 | P 5,66 |
| gef: | C 54,71 | H 9,31 | N 2,49 | P 5,39 |

3-Octadecyl-2-methyl-sn-glycero-3-phosphoserin, Natriumsalz

($C_{25}H_{51}NNaO_8P$; 547,66)

| | | | | |
|---|---|---|---|---|
| ber: | C 54,83 | H 9,39 | N 2,56 | P 5,66 |
| gef: | C 54,59 | H 9,24 | N 2,41 | P 5,43 |

Phosphatidylamide $VIII_{11}$ - $X_{11}$:

Die THF-Lösungen V - VII (0,1 Mol in 400 ml THF) werden bei 20°C tropfenweise unter Rühren mit einer Lösung von Triäthylamin (0,2 Mol) in 100 ml THF versetzt. Im Anschluß daran tropft man Bis-(chloräthyl)-amin (0,15 Mol) in 300 ml THF ein. Nach Beendigung der Reaktion (DC-Kontrolle) wird vom ausgefallenen Triäthylaminhydrochlorid abfiltriert und das Filtrat durch Zusatz von 100 ml Wasser hydrolysiert. Nach Beendigung der Hydrolyse wird mit 400 ml Chloroform extrahiert und die Chloroformphase noch mit 400 ml einer 0,5 M Natriumhydrogencarbonatlösung gewaschen. Der Rückstand wird an Kieselgel chromatographiert. Man erhält die gewünschten Produkte, $VIII_{11}$ - $X_{11}$, in ca. 80 % Ausbeute.

Verbindungen der Formeln $VIII_{11}$ - $X_{11}$:

1-Octadecyl-2-methyl-sn-glycero-3-phospho-bis-(chloräthyl)-amid

($C_{26}H_{53}Cl_2NNaO_5P$; 584,60)

| | | | | |
|---|---|---|---|---|
| ber: | C 53,42 | H 9,14 | N 2,40 | P 5,30 |
| gef: | C 53,21 | H 9,08 | N 2,26 | P 6,16 |

## Patentansprüche

1. D-Mannitderivate der allgemeinen Formel I

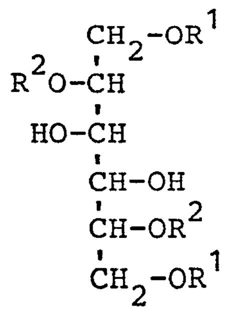

(I)

worin $R^1$ und $R^2$ gleich oder verschieden sind und, wenn $R^1$ und $R^2$ gleich sind, eine geradkettige oder verzweigte Alkyl-, Alkenyl- oder Alkinylgruppe mit 6 bis 24 Kohlenstoffatomen bedeuten, die Cycloalkylreste mit 3 bis 6 Kohlenstoffatomen, Arylreste, Benzyloxy-, Allyloxy-, Mesyloxy- und/oder Halogensubstituenten enthalten können, und, wenn $R^1$ und $R^2$ verschieden sind, eine geradkettige oder verzweigte Alkylgruppe mit 1 bis 24 Kohlenstoffatomen, die Cycloalkylreste mit 3 bis 6 Kohlenstoffatomen, Arylreste , Benzyloxy-, Allyloxy-, Mesyloxy- und/oder Halogensubstituenten enthalten können, oder eine geradkettige oder verzweigte Alkenyl- oder Alkinylgruppe mit 3 bis 24 Kohlenstoffatomen, die Cycloalkylreste mit 3 bis 6 Kohlenstoffatomen, Arylreste, Benzyloxy-, Allyloxy-, Mesyloxy- und/oder Halogensubstituenten enthalten können, bedeuten, und $R^1$ auch Trityl sein kann.

2. D-Mannitderivate nach Anspruch 1, dadurch gekennzeichnet, daß mindestens ein Rest $R^1$ oder $R^2$ mehr als 6 Kohlenstoffatome, insbesondere mehr als 9 Kohlenstoffatome, besitzt.

3. D-Mannitderivate nach Anspruch 1 oder 2, dadurch gekennzeichnet, daß sie 1,6-Dibenzyl-2,5-diallyl-D-mannit, 1,6-Diallyl-2,5-dibenzyl-D-mannit, 1,6-Ditrityl-2,5-dibenzyl-D-mannit, 1,6-Ditrityl-2,5-diallyl-D-mannit, 1,6-Ditrityl-2,5-dialkyl-D-mannit, 1,6-Ditrityl-2,5-dialkenyl-D-mannit, 1,6-Ditrityl-2,5-dialkinyl-D-mannit, 1,6-Dialkyl-2,5-dibenzyl-D-mannit, 1,6-Dialkyl-2,5-diallyl-D-mannit, 1,6-Dialkyl-2,5-dialkyl-D-mannit, 1,6-Dialkenyl-2,5-dialkyl-D-mannit, 1,6-Dialkinyl-2,5-dialkyl-D-mannit, 1,6-Dialkyl-2,5-dialkenyl-D-mannit, 1,6-Dialkenyl-2,5-dialkenyl-D-mannit, 1,6-Dialkinyl-2,5-dialkenyl-D-mannit, 1,6-Dialkyl-2,5-dialkinyl-D-mannit, 1,6-Dialkenyl-2,5-dialkinyl-D-mannit, 1,6-Dialkinyl-2,5-dialkinyl-D-mannit sind.

4. D-Mannitderivate nach Anspruch 1 oder 2, dadurch gekennzeichnet, daß sie 1,6-Dioctadecyl-2,5-dimethyl-D-mannit, 1,6-Dimethyl-2,5-dioctadecyl-D-mannit, 1,6-Dioctadecyl-2,5-diallyl-D-mannit, 1,6-Dioctadecyl-2,5-dibenzyl-D-mannit, 1,6-Dioctadec-(3')-enyl-2,5-dimethyl-D-mannit, 1,6-Dioctadec-(3')-inyl-2,5-dimethyl-D-mannit, 1,6-Dioctadecyl-2,5-ditetradecyl-D-mannit, 1,6-Dioctadecyl-2,5-didodecyl-D-mannit, 1,6-Dioctadecyl-2,5-didecyl-D-mannit, 1,6-Dioctadecyl-2,5-dioctyl-D-mannit, 1,6-Dioctadecyl-2,5-dibutyl-D-mannit, 1,2,5,6-Tetraoctadecyl-D-mannit, 1,2,5,6-Tetrahexadecyl-D-mannit, 1,2,5,6-Tetra-(tetradecyl)-D-mannit, 1,6-Ditrityl-2,5-dioctadecyl-D-mannit, 1,6-Ditrityl-2,5-dihexadecyl-D-mannit, 1,6-Ditrityl-2,5-ditetradecyl-D-mannit, 1,6-Ditrityl-2,5-didodecyl-D-mannit, 1,6-Ditrityl-2,5-didecyl-D-mannit, 1,6-Ditrityl-2,5-dioctyl-D-mannit und 1,6-Ditrityl-2,5-dihexyl-D-mannit sind.

5. Verfahren zur Herstellung der D-Mannitderivate

der Formel I nach einem der Ansprüche 1 bis 4, dadurch gekennzeichnet, daß man 3,4-Isopropyliden-D-mannit zum 1,6-Ditrityl-3,4-isopropyliden-D-mannit trityliert, in die so erhaltene Verbindung die Gruppe $R^2$ durch Umsetzung mit dem entsprechenden $R^2$-Mesylat, -Chlorid, -Bromid oder -Jodid oder mit $(R^2O)_2SO_2$ einführt, danach für den Fall, daß $R^1$ nicht Trityl ist, die Tritylgruppe in schwach saurem Medium abspaltet, und in das enttritylierte Produkt die Gruppe $R^1$ durch Umsetzung mit dem entsprechenden $R^1$-Mesylat, -Chlorid, -Bromid oder -Jodid oder mit $(R^1O)_2SO_2$ einführt, und dann die Isopropylidengruppe abspaltet.

6. Glycerinderivate der allgemeinen Formeln II, III und IV

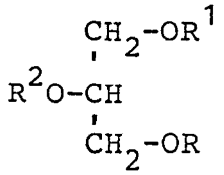

(II)

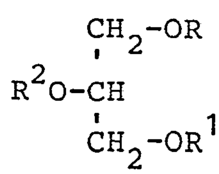

(III)

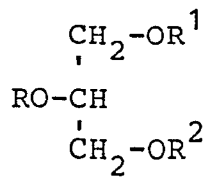

(IV)

worin $R^1$ und $R^2$ die in Anspruch 1 angegebenen Bedeutungen besitzen und R ein Wasserstoffatom oder den Rest

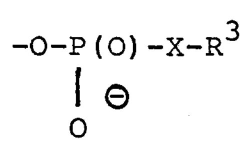

darstellt, worin X = O, NH oder $NR^3$ ist, und, wenn X = O ist, $R^3$ H, Alkyl, Alkenyl oder Alkinyl mit 1 bis 18 Kohlenstoffatomen, Halogenalkyl mit 2 bis 14 Kohlenstoffatomen, 2-Amino-2-carboxy-äthyl, Dihydroxypropyl, Pentahydroxyhexyl, Aminoalkyl mit 2 bis 14 Kohlenstoffatomen, N-Methyl-aminoalkyl mit 2 bis 14 Kohlenstoffatomen, N,N-Dimethyl-aminoalkyl mit 2 bis 14 Kohlenstoffatomen, N,N,N-Trimethyl-aminoalkyl mit 2 bis 14 Kohlenstoffatomen, N-/(N,'N,'N'-Trimethyl)-aminoäthyl7-N,N-dimethyl-aminoäthyl bedeutet, wenn X = NH ist, $R^3$ H, Alkyl oder Alkenyl mit 1 bis 10 Kohlenstoffatomen, Halogenalkyl mit 2 bis 6 Kohlenstoffatomen, Hydroxyäthyl, Dihydroxypropyl, Aminoalkyl bedeutet, und, wenn X = $NR^3$ ist, $R^3$ Alkyl, Alkenyl mit 1 bis 10 Kohlenstoffatomen, Bis-chloräthyl bedeutet, und wobei, wenn R kein Wasserstoffatom ist, einer der Reste $R^1$ oder $R^2$ in Formel II, III oder IV auch ein Wasserstoffatom sein kann.

7. Verfahren zur Weiterverarbeitung der D-Mannitderivate nach einem der Ansprüche 1 bis 4 zur Herstellung der Glycerinderivate der Formeln II, III oder IV nach Anspruch 6, dadurch gekennzeichnet, daß man

a) zur Herstellung der Verbindungen der Formel II die D-Mannitderivate der Formel I mit Bleitetraacetat umsetzt und in dem entstandenen Glycerinaldehydderivat die CHO-Gruppe zur $CH_2OH$-Gruppe reduziert,

b) zur Herstellung der Verbindungen der Formel III von D-Mannitderivaten der Formel I ausgeht, in denen $R^1$ eine Tritylgruppe, oder, wenn $R^1$ in Formel III gesättigt ist, auch eine Benzylgruppe ist, mit Bleitetraacetat umsetzt, in dem entstandenen Glycerinaldehydderivat die CHO-Gruppe zur $CH_2OH$-Gruppe reduziert, in die so erhaltene Verbindung die Gruppe $R^1$ durch Umsetzung mit dem entsprechenden $R^1$-Mesylat, -Chlorid, -Bromid oder -Jodid oder mit $(R^1O)_2SO_2$ einführt und danach die Tritylgruppe durch saure Hydrolyse oder die Benzylgruppe durch katalytische Hydrogenolyse abspaltet,

c) zur Herstellung von Verbindungen der Formel IV von D-Mannitderivaten der Formel I ausgeht, in denen $R^1$ eine Alkylgruppe und $R^2$ eine Allylgruppe oder, wenn $R^1$ und $R^2$ in Formel IV gesättigt sind, auch eine Benzylgruppe ist, mit Bleitetraacetat umsetzt, in dem entstandenen Glycerinaldehydderivat die CHO-Gruppe zur $CH_2OH$-Gruppe reduziert, in der so erhaltenen Verbindung gegebenenfalls die Allylgruppe in eine Propenylgruppe umlagert, $R^2$ durch Umsetzung mit dem entsprechenden $R^2$-Mesylat, -Chlorid, -Bromid oder -Jodid oder mit $(R^2O)_2SO_2$ einführt und danach die Propenylgruppe durch saure Hydrolyse oder die Benzylgruppe durch katalytische Hydrogenolyse wieder abspaltet, und wenn erwünscht

in die nach a), b) oder c) erhaltenen Verbindungen, in denen R ein Wasserstoffatom bedeutet, den Rest

$$-O-\underset{\displaystyle O^{\ominus}}{\underset{|}{P}}(O)-X-R^3$$

auf an sich bekannte Weise einführt, und, wenn erwünscht, in einer Verbindung der Formel II, III oder IV, in der R kein Wasserstoffatom bedeutet, einen Benzyl- oder Allylrest $R^1$ oder $R^2$ auf an sich bekannte Weise in ein Wasserstoffatom überführt.

8. Verfahren nach Anspruch 7, dadurch gekennzeichnet, daß man von einem nach einem der Verfahrensstufen erhaltenen Produkt ausgeht und die restlichen Verfahrensstufen durchführt.

9. Arzneimittel, enthaltend eine oder mehrere der Verbindungen der Formeln II bis IV nach Anspruch 6, in denen R den Rest

$$-O-\underset{\substack{|\\ O^{\ominus}}}{P}(O)-X-R^3$$

bedeutet.